# EUROPEAN PATENT APPLICATION

(11) **EP 3 992 284 A1**
(43) Date of publication of application: **04.05.2022**
(21) Application number: 20832476.4
(22) Date of filing: 25.06.2020
(51) Int. Cl.: C12N 9/10, C12Q 1/6883, A61P 13/12

(54) **NOVEL ADP-RIBOSYL CYCLASE AND INHIBITOR THEREOF**

(30) Priority: 27.06.2019 KR 20190076979; 23.06.2020 KR 20200076635
(71) Applicant: Industrial Cooperation Foundation Chonbuk National University, Jeonju-si, Jeollabuk-do 54896 (KR); DNT Co. Ltd., Jeonju-si, Jeollabuk-do 54969 (KR)
(72) Inventor: KIM, Uh-Hyun, Jeonju-si, Jeollabuk-do 54823 (KR); NAM, Tae-Sik, Jeonju-si, Jeollabuk-do 55028 (KR)
(74) Representative: V.O.
(86) International application number: PCT/KR2020/008283
(87) International publication number: WO 2020/262983

(57) **Abstract**

The present disclosure relates to a pharmaceutical composition containing an inhibitor against the expression or activation of a novel ADP-ribosyl cyclase or a naturally occurring variant thereof as an active ingredient for preventing or treating an ADP-ribosyl cyclase-mediated disease. In addition, the present disclosure relates to a composition for diagnosis of an ADP-ribosyl cyclase-mediated disease, the composition containing an agent for measuring a gene expression level or protein level of the ADP-ribosyl cyclase or a naturally occurring variant thereof. The composition of the present disclosure has the effect of inhibiting calcium increase in kidney cells, which is attributed to angiotensin II-induced ADP-ribosyl cyclase expression or activation, and as such can be advantageously used as a therapeutic agent for an ADP-ribosyl cyclase-mediated disease, particularly a renal disease.

## Description

### [Technical Field]

The present disclosure relates to a novel ADP-ribosyl cyclase which converts NAD⁺ to cyclic ADP-ribose and an inhibitor thereof.

### [Background Art]

ADPRC is an enzyme which synthesizes cyclic ADP-ribose (cADPR) from NAD⁺ and synthesizes nicotinic acid adenine dinucleotide phosphate (NAADP) from NADP⁺. It exists widely in from plants to mammals. The ADPRC is known to regulate various cellular functions by releasing calcium from intracellular stores [Berridge MJ, Bootman MD, Roderick HL. Nat Rev Mol Cell Biol. 4, 517-529, 2003; Lee HC, Mol. Med. 12, 317-323, 2006].

In stable state, the intracellular calcium concentration is maintained at a baseline of 10⁻⁷ M or lower. However, in activated state, the intracellular calcium concentration is increased up to 10 times of the baseline. In pathological state, where the calcium concentration is maintained high continuously, many cellular functions are affected and, as a result, the normal functions of cells are lost.

In hypertension or diseases accompanying hypertension such as diabetes, obesity, dementia, ischemia or cellular proliferation, the intracellular calcium concentration is maintained above a normal level due to anomaly in the regulation of intracellular calcium metabolism [Resnick LM. Am. J. Hypertens. 6: 123S-134S, 1993].

For instance, patients with chronic diseases such as hypertension show high levels of intracellular calcium concentration in vascular smooth muscle because of various hereditary, environmental or secondary causes of some diseases. As a result, peripheral vascular resistance is increased due to the contraction of the vascular smooth muscle, which causes hypertension by maintaining blood pressure consistently higher than the normal level.

It is known that the increased blood pressure secondarily causes the proliferation and hypertrophy of vascular smooth muscle cells and fibrous tissues, which further increases peripheral vascular resistance and blood pressure, leading to myocardial infarction, angina, palsy, chronic heart failure, and even death due to the dysfunction of the cardiovascular system, brain, kidney, etc. [Cowley AW Jr. Physiol Rev. 72: 231-300, 1992].

It is known that the calcium increase by the activation of CD38, which is a type of ADPRC, i.e., cADPR, plays an important role in insulin secretion from the pancreas. In addition, the induction of diabetes increases the production of angiotensin. It is well known that this peptide hormone not only induces renal diseases, hypertension and heart diseases but also activates ADPRC.

Existing in immune cells, cardiomyocytes, pancreatic beta cells, extensor muscle cells, neurons, etc., ADPRC increases intracellular calcium concentration and regulates several physiological activities in association with the receptors of several hormones. The dysregulation of the expression or activation of ADPRC may cause abnormalities in the regulation of physiological phenomena (immunity, renal function, insulin secretion and cardiovascular function).

ADPRC is activated by several hormones. This enzyme produces cADPR from the substrate NAD⁺. The cADPR increases intracellular calcium level in almost all organs. In addition, abnormal increase in calcium owing to activation of ADPRC has been known as the cause of various pathologies such as insulin secretion [Kim BJ, Park KH, Yim CY, Takasawa S, Okamoto H, Im MJ, Kim UH. Diabetes. 57: 868-878, 2008], cellular hypertrophy [Gul R, Park JH, Kim SY, Jang KY, Chea JK, Ko JK, Kim UH. Cardiovasc Res. 81: 582-591, 2009], cell proliferation [Kim SY, Gul R, Rah SY, Kim SH, Park SK, Im MJ, Kwon HJ, Kim UH. Am J Physiol Renal Physiol. 294: F989-F989, 2008], etc.

The ADPRC that has been studied best thus far is the T-cell surface antigen CD38. This molecule is expressed widely not only in immune cells but also in many organs.

However, it has been found out that ADPRCs different from CD38 are expressed in the heart, kidney and brain [Partida-Sanchez S, Cockayne DA, Monard S, Jacobson EL, Oppenheimer N, Garvy B, Kusser K, Goodrich S, Howard M, Harmsen A, Randall TD, Lund FE. Nat Med 7: 1209-16, 2001].

Accordingly, the regulation of tissue-specific ADPRC expression will be helpful in treating a disease induced by increased intracellular calcium level. The activation mechanism of ADPRC is not known well yet.

The foregoing description in the Background section is only for enhancing the understanding about the background of the present disclosure, and it should not be regarded as admitting that it is well known to those having ordinary knowledge in the art.

### [Disclosure]

### [Technical Problem]

The inventors of the present disclosure have found a new type of ADPRC other than the existing ADPRCs known in mammals (CD38 and CD157), identified its activity and completed the present disclosure.

The present disclosure is directed to providing a novel ADP-ribosyl cyclase (ADPRC) or a naturally occurring variant thereof.

The present disclosure is also directed to providing a nucleic acid molecule which encodes the ADP-ribosyl cyclase or a naturally occurring variant thereof.

The present disclosure is also directed to providing a vector including the nucleic acid molecule.

The present disclosure is also directed to providing a host cell including the vector.

The present disclosure is also directed to providing a catalyst composition converting NAD⁺ to cyclic ADP-ribose (cADPR), which contains the ADP-ribosyl cyclase or a naturally occurring variant thereof, a nucleic acid molecule encoding the same or a vector including the nucleic acid molecule.

The present disclosure is also directed to providing a pharmaceutical composition for preventing or treating an ADP-ribosyl cyclase-mediated disease, which contains an inhibitor against the expression or activation of the novel ADP-ribosyl cyclase or a naturally occurring variant thereof as an active ingredient.

The present disclosure is also directed to providing a food composition for preventing or alleviating an ADP-ribosyl cyclase-mediated disease, which contains an inhibitor against the expression or activation of the novel ADP-ribosyl cyclase or a naturally occurring variant thereof as an active ingredient.

The present disclosure is also directed to providing an animal model wherein a hetero-type gene of the ADP-ribosyl cyclase or a naturally occurring variant thereof is deleted.

The present disclosure is also directed to providing a method for providing information for diagnosis of an ADP-ribosyl cyclase-mediated disease.

The present disclosure is also directed to providing a composition for diagnosis of an ADP-ribosyl cyclase-mediated disease, which contains an agent for measuring a gene expression level or protein level of the ADP-ribosyl cyclase or a naturally occurring variant thereof.

The present disclosure is also directed to providing a kit for diagnosis of an ADP-ribosyl cyclase-mediated disease, which includes an agent for measuring a gene expression level or protein level of the ADP-ribosyl cyclase or a naturally occurring variant thereof.

The present disclosure is also directed to providing a method for screening a substance for preventing or treating the ADP-ribosyl cyclase-mediated disease.

Other purposes and advantages of the present disclosure will become more apparent by the following detailed description, claims and drawings.

### [Technical Solution]

In an aspect, the present disclosure provides an ADP-ribosyl cyclase (ADPRC) including an amino acid sequence of SEQ ID NO 1 or a naturally occurring variant thereof.

The inventors of the present disclosure have made consistent efforts to discover a novel ADP-ribosyl cyclase other than the previously known ADP-ribosyl cyclases, as an enzyme which converts NAD⁺ to cyclic ADP-ribose (cADPR), and have completed the present disclosure as a result.

In the present specification, the term "ADP-ribosyl cyclase" or "ADP-ribosyl cyclase (ADPRC)" refers to an enzyme which converts NAD⁺ to cyclic ADP-ribose (cADPR).

In the present specification, the term "variant of ADP-ribosyl cyclase" refers to a variant which is obtained from natural or artificial substitution, deletion or addition of a part of the amino acid sequence of the ADP-ribosyl cyclase and retains the catalytic activity of the ADP-ribosyl cyclase of converting NAD⁺ to cyclic ADP-ribose (cADPR).

In the present specification, the term "naturally occurring variant of ADP-ribosyl cyclase" refers to a naturally occurring variant of the ADP-ribosyl cyclase including the amino acid sequence of SEQ ID NO 1, which retains the catalytic activity of converting NAD⁺ to cyclic ADP-ribose (cADPR).

In a specific exemplary embodiment of the present disclosure, the naturally occurring variant of ADP-ribosyl cyclase of the present disclosure is a naturally occurring variant selected from a group consisting of an interspecies variant, a species homolog, an isoform, an allelic variant, a conformational variant, a splice variant and a point mutation variant.

The naturally occurring variant of ADP-ribosyl cyclase of the present disclosure has specifically 50% or higher homology, more specifically 60% or higher homology, further more specifically 70% or higher homology, even more specifically 80% or higher homology, most specifically 90% or higher homology, to ADP-ribosyl cyclase including the amino acid sequence of SEQ ID NO 1.

In a specific exemplary embodiment of the present disclosure, the naturally occurring variant of ADP-ribosyl cyclase of the present disclosure originates from an organism selected from a group consisting of mammals, birds, reptiles, amphibians and fish.

In an example of the present disclosure, it was confirmed that the naturally occurring variant of ADP-ribosyl cyclase of the present disclosure has 70% or higher homology for mammals, 60% or higher homology for birds, reptiles and amphibians and 50% or higher homology for fish, to the ADP-ribosyl cyclase including the amino acid sequence of SEQ ID NO 1.

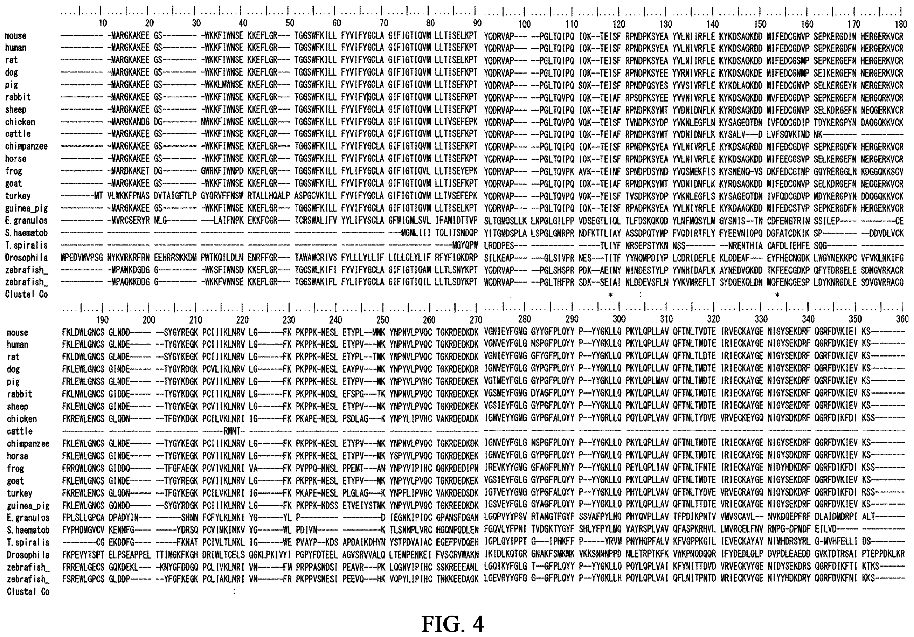

In an example of the present disclosure, it was confirmed that the naturally occurring variant of ADP-ribosyl cyclase of the present disclosure is an enzyme exhibiting a very high proportion of interspecies conserved sequences with 96% homology for human and rat, 93% homology for chimpanzee, 91% homology for guinea pig and horse, 90% homology for dog, goat and sheep, 89% homology for rabbit, 88% homology for pig, 78% homology for cattle, 70% homology for chicken, 63% homology for frog and 62% homology for turkey, to the ADP-ribosyl cyclase including the amino acid sequence of SEQ ID NO 1 (FIG. 4).

In a specific exemplary embodiment of the present disclosure, the naturally occurring variant of ADP-ribosyl cyclase of the present disclosure includes an amino acid sequence selected from a group consisting of SEQ ID NOS 2-21.

In another aspect, the present disclosure provides a nucleic acid molecule encoding the ADP-ribosyl cyclase or naturally occurring variant thereof, a vector including the nucleic acid molecule or a host cell including the vector.

The nucleic acid molecule of the present disclosure may be an isolated or recombinant nucleic acid molecule, and includes not only a single-stranded or double-stranded DNA or RNA but also a sequence complementary thereto. When the "isolated nucleic acid" is a nucleic acid isolated from a natural origin, the nucleic acid is a nucleic acid separated from nearby gene sequences existing in the isolated genome of an individual. For nucleic acids, e.g., PCR products, cDNA molecules or oligonucleotides, synthesized enzymatically or chemically from a template, the nucleic acids produced from these procedures may be understood as isolated nucleic acid molecules. An isolated nucleic acid molecule may be a component of a fragment or a larger nucleic acid construct. A nucleic acid is "operably linked" when it is placed into a functional relationship with another nucleic acid sequence. For example, a DNA for a presequence or a secretory leader is operably linked to a DNA for a polypeptide if it is expressed as a preprotein that participates in the secretion of the polypeptide. A promoter or an enhancer is operably linked to a coding sequence if it affects the transcription of the polypeptide sequence. And, a ribosome-binding site is operably linked to a coding sequence if it is positioned to facilitate translation. In general, "operably linked" means that the DNA sequences being linked are contiguous and, in the case of a secretory leader, contiguous and present in the same reading frame. However, enhancers do not have to be contiguous. The linking is accomplished by ligation at convenient restriction enzyme sites. If such sites do not exist, synthetic oligonucleotide adaptors or linkers are used in accordance with common methods.

In a specific exemplary embodiment of the present disclosure, the nucleic acid molecule is a cDNA.

In the present specification, the term "vector" refers to a carrier capable of inserting a nucleic acid sequence for introduction into a cell capable of replicating the nucleic acid sequence. The nucleic acid sequence may be exogenous or heterologous. The vector may be a plasmid, a cosmid or a virus (e.g., a bacteriophage), although not being limited thereto. Those skilled in the art can construct the vector according to the standard recombination technology (Maniatis, et al., Molecular Cloning, A Laboratory Manual, Cold Spring Harbor Press, Cold Spring Harbor, N.Y., 1988; Ausubel et al., In: Current Protocols in Molecular Biology, John, Wiley & Sons, Inc., NY, 1994, etc.).

In the present specification, the term "expression vector" refers to a vector including a nucleic acid sequence encoding at least a part of a transcribed gene product. In some cases, an RNA molecule is post-translated into a protein, a polypeptide or a peptide. The expression vector may contain various regulatory sequences. A vector or an expression vector may further contain a nucleic acid sequence that provides another function, in addition to a regulatory sequence regulating transcription and translation.

In the present specification, the term "host cell" refers to a cell of any transformable organism, including a eukaryote and a prokaryote, which is capable of replicating the vector or expressing a gene encoded by the vector. The host cell may be transfected or transformed by the vector, which means a process by which an exogenous nucleic acid molecule is delivered or introduced into the host cell.

The host cell of the present disclosure may be specifically a bacterial cell, a yeast cell, an animal cell or a human cell (CHO cell, HeLa cell, HEK293 cell, MES13 cell, BHK-21 cell, COS7 cell, COP5 cell, A549 cell, NIH3T3 cell, etc.), although not being limited thereto.

In another aspect, the present disclosure provides a catalyst composition which converts NAD⁺ to cyclic ADP-ribose (cADPR), which contains the ADP-ribosyl cyclase or naturally occurring variant thereof, the nucleic acid molecule encoding the same or the vector including the nucleic acid molecule.

The catalyst composition of the present disclosure effectively catalyzes the process of converting NAD⁺ to cADPR.

In another aspect, the present disclosure provides a composition containing an inhibitor against the expression or activation of the novel ADP-ribosyl cyclase or a naturally occurring variant thereof.

In a specific exemplary embodiment of the present disclosure, the composition of the present disclosure is a pharmaceutical composition for preventing or treating an ADP-ribosyl cyclase-mediated disease.

The pharmaceutical composition of the present disclosure may contain: (a) the inhibitor against the expression or activation; and (b) a pharmaceutically acceptable carrier.

In a specific exemplary embodiment of the present disclosure, the composition of the present disclosure is a food composition for preventing or alleviating an ADP-ribosyl cyclase-mediated disease.

In another aspect, the present disclosure provides a method for preventing or treating an ADP-ribosyl cyclase-mediated disease, which includes a step of administering a pharmaceutically effective amount of the pharmaceutical composition to a subject.

In another aspect, the present disclosure provides an inhibitor against the expression or activation of the novel ADP-ribosyl cyclase or a naturally occurring variant thereof for use in therapy.

The ADP-ribosyl cyclase-mediated disease to be prevented or treated is not limited specially. The disease may be specifically diabetes or renal disease, more specifically renal failure, nephropathy, nephritis, renal fibrosis or nephrosclerosis.

In a specific exemplary embodiment of the present disclosure, the renal failure may be chronic renal failure, acute renal failure or mild renal failure before dialysis.

In a specific exemplary embodiment of the present disclosure, the nephropathy may be nephropathy syndrome, lipoid nephropathy, diabetic nephropathy, immunoglobulin A (IgA) nephropathy, analgesic nephropathy or hypertensive nephropathy.

In an example of the present disclosure, it was confirmed that the composition of the present disclosure is a good candidate as a therapeutic agent for renal diseases including chronic renal failure or diabetic nephropathy through significant decrease in kidney to body weight, significant increase in creatinine clearance rate and significant decrease in urine albumin (FIG. 6). In addition, it was confirmed that it lowers the activity of ADP-ribosyl cyclase and the concentration of cADPR to normal levels (FIG. 7), lowers the expression levels of TGF-β1, fibronectin and collagen IV to normal levels (FIG. 8), and recovers the histopathological change of the kidney by recovering glomerulus hypertrophy, infiltration of inflammatory cells and formation of transitional epithelial cells to normal levels (FIG. 9). Furthermore, it was confirmed that it can be applied to hypertensive nephropathy by significantly increasing creatinine clearance rate in a hypertension model (FIG. 11B). Therefore, the composition can be used as a therapeutic agent for various renal diseases caused by the failure or loss of kidney function.

In the present specification, the term "inhibitor of expression" refers to a substance which inhibits the expression of the ADP-ribosyl cyclase, and it may be easily prepared by those having ordinary skill in consideration of the structure and function of the ADP-ribosyl cyclase.

The inhibitor of expression of the present disclosure is specifically any one selected from a group consisting of an antisense oligonucleotide, a siRNA, a shRNA, a miRNA, a ribozyme, a DNAzyme and a PNA (protein nucleic acid) binding complementarily to the mRNA of the ADP-ribosyl cyclase or naturally occurring variant thereof, although not being limited thereto.

In a specific exemplary embodiment of the present disclosure, the siRNA includes a nucleotide sequence of SEQ ID NO 22.

In the present specification, the term "inhibitor of activation" refers to a substance which inhibits the activation of the expressed ADP-ribosyl cyclase protein, and it is specifically any one selected from a group consisting of a compound, a peptide, a peptide mimetic, an aptamer and an antibody binding specifically to the ADP-ribosyl cyclase or naturally occurring variant thereof protein.

In a specific exemplary embodiment of the present disclosure, the compound is selected from a group consisting of 4,4'-dihydroxyazobenzene, 2-(1,3-benzoxazol-2-ylamino)-1-methylquinazolin-4(1H)-one and dicaffeoylquinic acid (DCQA).

Specifically, the 4,4'-dihydroxyazobenzene of the present disclosure may be represented by Chemical Formula 1:

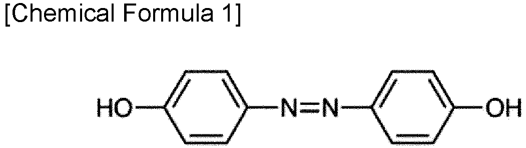

Specifically, the 2-(1,3-benzoxazol-2-ylamino)-1-methylquinazolin-4(1H)-one of the present disclosure may be represented by Chemical Formula 2:

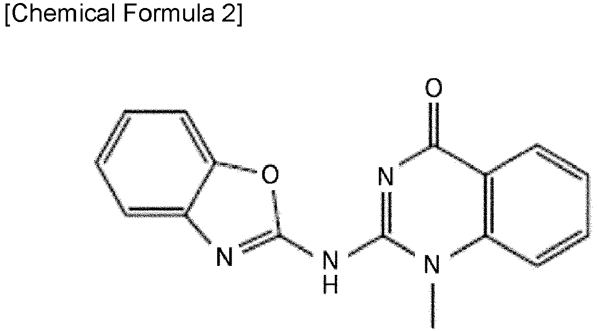

The dicaffeoylquinic acid (DCQA) of the present disclosure may be any one selected from a group consisting of the compounds represented by Chemical Formulas 3-8 (1,4-DCQA, 3,4-DCQA, 3,5-DCQA, 4,5-DCQA, 1,3-DCQA and 1,5-DCQA):

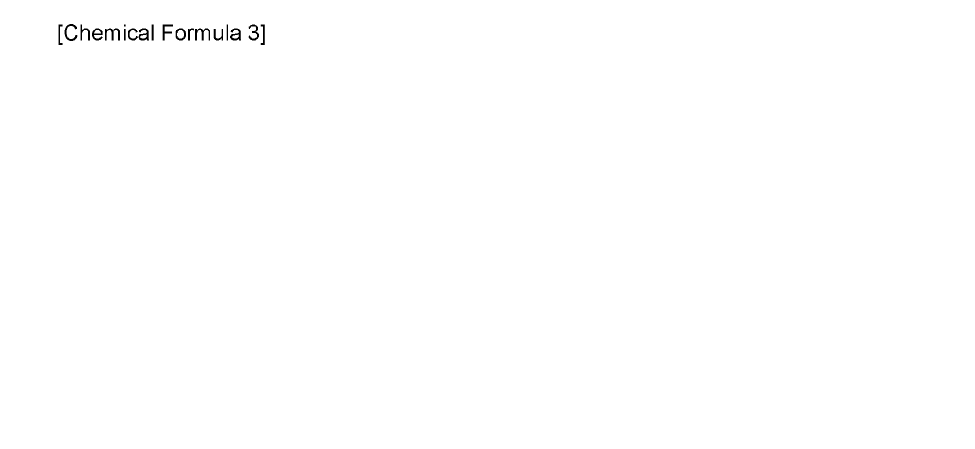

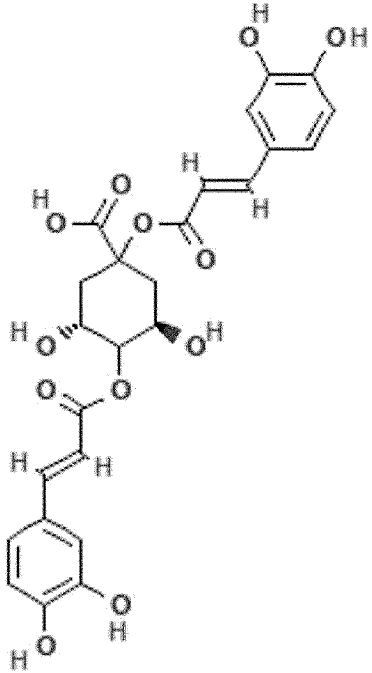

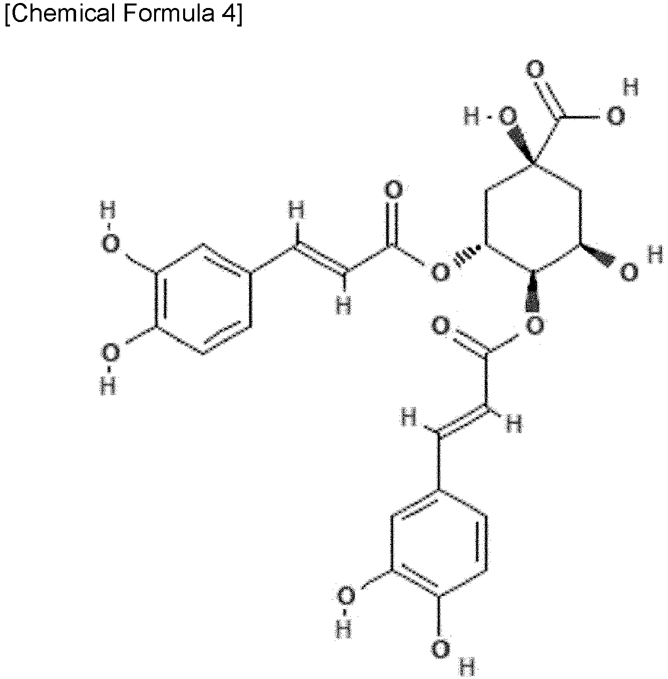

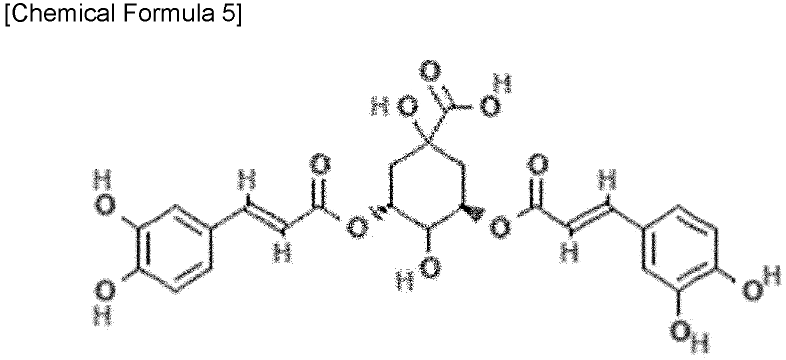

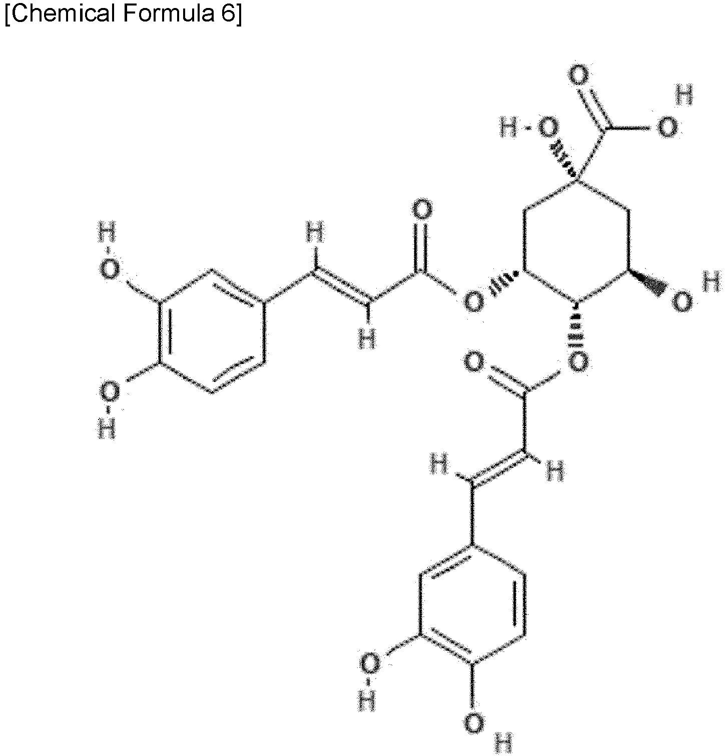

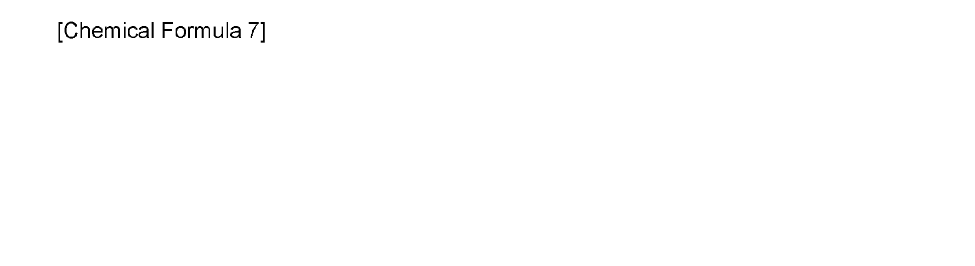

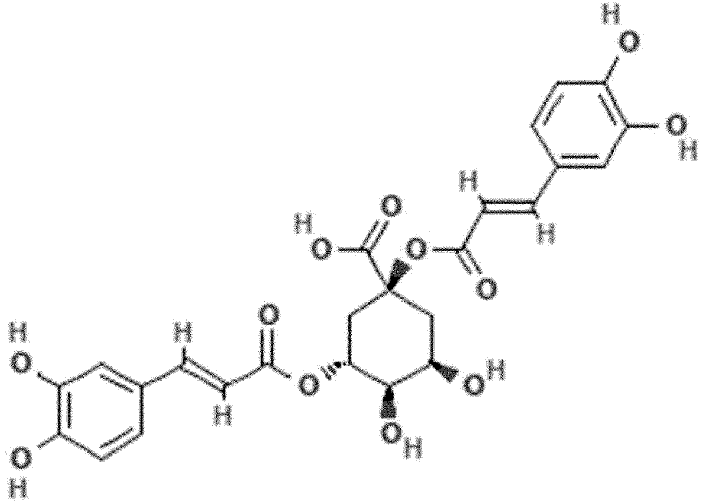

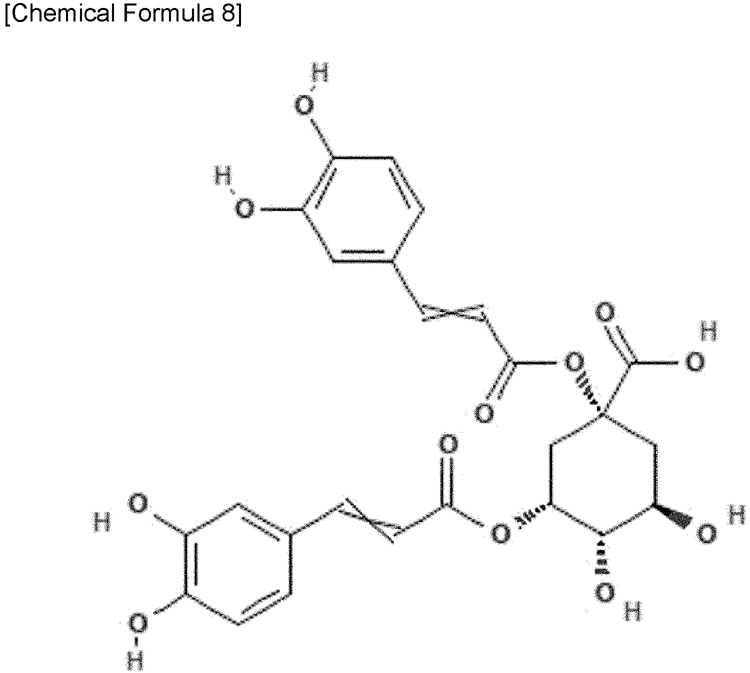

In an example of the present disclosure, it was confirmed that the inhibitor against the expression or activation of the novel ADP-ribosyl cyclase according to the present disclosure contributes to the inhibition of a renal disease via a mechanism of regulating the expression or activation of ADPRC.

Accordingly, the inhibitor against the expression or activation of the novel ADP-ribosyl cyclase of the present disclosure may be used as a clinically useful selective inhibitor for individual cells and, furthermore, as an agent for preventing, alleviating and/or treating a renal disease.

The composition of the present disclosure may contain, in addition to the inhibitor against the expression or activation of the novel ADPRC as an active ingredient, other previously known therapeutic agents for ADPRC-related diseases.

The pharmaceutical composition of the present disclosure may be formulated into a suitable form together with a pharmaceutically acceptable carrier. 'Pharmaceutically acceptable' means that the carrier which is physiologically acceptable and does not cause allergic reactions such as gastrointestinal disturbance, vertigo, etc. and similar responses.

The pharmaceutically acceptable carrier contained in the pharmaceutical composition of the present disclosure includes, as those commonly used in formulation, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc., although not being limited thereto. In addition to the above-described ingredients, the pharmaceutical composition of the present disclosure may further contain a lubricant, a wetting agent, a sweetener, a flavorant, an emulsifier, a suspending agent, a preservative, etc. Suitable pharmaceutically acceptable carriers and formulations are described in detail in Remington's Pharmaceutical Sciences (19th ed., 1995).

The pharmaceutical composition of the present disclosure may be administered orally or parenterally. Specifically, it may be administered parenterally, e.g., intravenously, topically, intraperitoneally, etc.

An appropriate administration dosage of the pharmaceutical composition of the present disclosure varies depending on such factors as formulation method, administration method, the age, body weight, sex, pathological condition and diet of a patient, administration time, administration route, excretion rate and response sensitivity, and an administration dosage effective for the desired treatment or prevention may be easily determined by an ordinarily skilled physician. In a specific exemplary embodiment of the present disclosure, a daily administration dosage of the pharmaceutical composition of the present disclosure is 0.0001-100 mg/kg.

The pharmaceutical composition of the present disclosure may be prepared as a single-dose or multiple-dose formulation using a pharmaceutically acceptable carrier and/or excipient according to a method that can be easily carried out by those having ordinary knowledge in the art. The formulation may be in the form of a solution in an oily or aqueous medium, a suspension, an emulsion, an extract, a powder, a granule, a tablet or a capsule, and may further contain a dispersant or a stabilizer.

When the composition of the present disclosure is prepared as a food composition, it may further contain ingredients commonly added for preparation of food in addition to the inhibitor against the expression or activation of the ADP-ribosyl cyclase as an active ingredient. For example, it may contain a protein, a carbohydrate, a fat, a nutrient, a condiment and a flavorant. The examples of the carbohydrate may include common sugars such as a monosaccharide, e.g., glucose, fructose, etc., a disaccharide, e.g., maltose, sucrose, oligosaccharides, etc., a polysaccharide, e.g., dextrin, cyclodextrin, etc., and sugar alcohols such as xylitol, sorbitol, erythritol, etc. As the flavorant, a natural flavorant (thaumatin, stevia extract [e.g., rebaudioside A, glycyrrhizin, etc.]) or a synthetic flavorant (saccharine, aspartame, etc.) may be used.

For example, when the food composition of the present disclosure is prepared as a drink, citric acid, fructose syrup, sugar, glucose, acetic acid, malic acid, fruit juice, eucommiae cortex extract, jujube extract, licorice extract, etc. may be further added in addition to the inhibitor against the expression or activation of ADP-ribosyl cyclase of the present disclosure.

The food composition of the present disclosure exhibits very superior effect in alleviating renal diseases through regulation of various kidney-related pathological factors (significant decrease in kidney to body weight, significant increase in creatinine clearance rate, significant decrease in urine albumin level, decrease in expression levels of TGF-β1, fibronectin and collagen IV to normal levels, and recovery of glomerulus hypertrophy, infiltration of inflammatory cells and formation of transitional epithelial cells to normal levels).

In another aspect, the present disclosure provides an animal model wherein a hetero-type gene of ADP-ribosyl cyclase (ADPRC) or a naturally occurring variant thereof is deleted.

In a specific exemplary embodiment of the present disclosure, the animal may be a non-human mammal, a bird, a reptile, an amphibian or a fish.

In another aspect, the present disclosure provides a method for identifying an ADP-ribosyl cyclase-mediated disease, which includes: (a) a step of inducing a specific disease in the animal model wherein a hetero-type gene of ADP-ribosyl cyclase (ADPRC) or a naturally occurring variant thereof is deleted and a wild-type animal model; and (b) a step of identifying the difference between the animal models.

By using the animal models, it is possible to identify whether a specific disease (e.g., diabetes, renal disease, hypertension, obesity, etc.) has occurred as being mediated by ADP-ribosyl cyclase or regardless of ADP-ribosyl cyclase based on the difference between the animal models (e.g., difference in body weight, kidney weight, blood pressure, creatinine clearance rate, blood sugar, inflammation, degree of glomerulus hypertrophy, etc.).

In another aspect, the present disclosure provides a method for providing information for diagnosis of an ADP-ribosyl cyclase-mediated disease, which includes:
1) a step of measuring the expression or activation level of an ADP-ribosyl cyclase including an amino acid sequence of SEQ ID NO 1 or a naturally occurring variant thereof in a sample isolated from a subject; and
2) a step of determining a risk of the ADP-ribosyl cyclase-mediated disease of the subject by comparing the expression or activation level of the ADP-ribosyl cyclase or naturally occurring variant thereof in the step 1) with a normal control group.

In the present specification, the term "diagnosis" refers to identification of the presence or characteristics of a pathological condition. In the purpose of the present disclosure, the diagnosis means the identification of the occurrence or the risk of occurrence of an ADP-ribosyl cyclase-related or mediated disease.

ADPRC is activated by several hormones. This enzyme produces cADPR from the substrate NAD⁺. The cADPR increases intracellular calcium level in almost all organs. In addition, abnormal increase in calcium owing to activation of ADPRC has been known as the cause of various pathologies such as insulin secretion, cellular hypertrophy, cell proliferation, etc. Accordingly, it is possible to provide useful information for diagnosis of the ADP-ribosyl cyclase-mediated disease induced by increased intracellular calcium level based on the measurement of the expression or activation level of the ADP-ribosyl cyclase.

In another aspect, the present disclosure provides a composition for diagnosis of an ADP-ribosyl cyclase-mediated disease, which contains an agent for measuring the gene expression level or protein level of an ADP-ribosyl cyclase including an amino acid sequence of SEQ ID NO 1 or a naturally occurring variant thereof.

In a specific exemplary embodiment of the present disclosure, the agent for measuring the gene expression level is a primer or a probe binding specifically to a gene encoding the ADP-ribosyl cyclase or naturally occurring variant thereof.

In a specific exemplary embodiment of the present disclosure, the agent for measuring the protein level is an antibody binding specifically to the ADP-ribosyl cyclase or naturally occurring variant thereof or an antigen-binding fragment thereof.

Since the antibody or antigen-binding fragment binds specifically to the ADP-ribosyl cyclase or naturally occurring variant thereof, it can be used to accurately measure the amount of the ADP-ribosyl cyclase or naturally occurring variant thereof contained in a sample.

The composition for diagnosis of the present disclosure may be used to quantify the amount of the ADP-ribosyl cyclase or naturally occurring variant thereof by analyzing an antigen for the antibody based on antigen-antibody binding reaction. Specifically, the analysis based on antigen-antibody binding reaction is selected from a group consisting of ELISA (enzyme-linked immunosorbent assay), RIA (radioimmunoassay), sandwich assay, western blot polyacrylamide gel, immunoblot assay and immunohistochemical staining, although not being limited thereto.

As a substrate for antigen-antibody binding reaction, one selected from a group consisting of a nitrocellulose membrane, a PVDF membrane, a well plate synthesized from a polyvinyl resin or a polystyrene resin and a slide glass may be used, although not being limited thereto.

Specifically, a secondary antibody may be labeled with a common color developing agent. A label selected from a group consisting of HRP (horseradish peroxidase), alkaline phosphatase, colloidal gold, a fluorescein such as FITC (poly-L-lysine fluorescein isothiocyanate), RITC (rhodamine B isothiocyanate), etc. and a dye may be used. Specifically, as a substrate inducing color development, any one selected from a group consisting of TMB (3,3',5,5'-tetramethylbezidine), ABTS [2,2'-azino-bis(3-ethylbenzothiazoline-6-sulfonic acid)] and OPD (o-phenylenediamine) may be used, although not being limited thereto.

In another aspect, the present disclosure provides a kit for diagnosis of an ADP-ribosyl cyclase-mediated disease, which includes an agent for measuring the gene expression level or protein level of the ADP-ribosyl cyclase including an amino acid sequence of SEQ ID NO 1 or a naturally occurring variant thereof.

The diagnostic kit of the present disclosure may further include one or more composition, solution or device suitable for analysis and an instruction thereabout.

In another aspect, the present disclosure provides a method for screening a substance for preventing or treating an ADP-ribosyl cyclase-mediated disease, which includes:
1) a step of treating a cell expressing an ADP-ribosyl cyclase including an amino acid sequence of SEQ ID NO 1 or a naturally occurring variant thereof with a test substance;
2) a step of measuring the gene expression level or protein level of the ADP-ribosyl cyclase or naturally occurring variant thereof as a result of treating with the test substance; and
3) a step of screening the test substance as a substance for preventing or treating an ADP-ribosyl cyclase-mediated disease if the gene expression level or protein level is decreased as compared to a control group not treated with the test substance.

Specifically, the gene expression level of the ADP-ribosyl cyclase or naturally occurring variant thereof may be measured by one or method selected from a group consisting of immunoprecipitation, radioimmunoassay (RIA), enzyme-linked immunosorbent assay (ELISA), immunohistochemistry, RT-PCR, western blot and fluorescence-activated cell sorting (FACS), although not being limited thereto.

Specifically, the protein level of the ADP-ribosyl cyclase or naturally occurring variant thereof may be measured by one or more method selected from a group consisting of SDS-PAGE, immunofluorescence, enzyme-linked immunosorbent assay (ELISA), mass spectrometry and protein chip assay, although not being limited thereto.

### [Advantageous Effects]

The features and advantages of the present disclosure may be summarized as follows:
(i) The present disclosure provides a pharmaceutical composition for preventing or treating an ADP-ribosyl cyclase-mediated disease, which contains an inhibitor against the expression or activation of a novel ADP-ribosyl cyclase or a naturally occurring variant thereof as an active ingredient.
(ii) The present disclosure also provides a composition for diagnosis of an ADP-ribosyl cyclase-mediated disease, which contains an agent for measuring a gene expression level or protein level of the ADP-ribosyl cyclase or a naturally occurring variant thereof.
(iii) The composition of the present disclosure may be usefully used as a therapeutic agent for an ADP-ribosyl cyclase-mediated disease, particularly a renal disease, because it is effective in inhibiting calcium increase in kidney cells by increasing the expression or activation of the novel ADP-ribosyl cyclase when stimulated with angiotensin II.

### [Brief Description of Drawings]

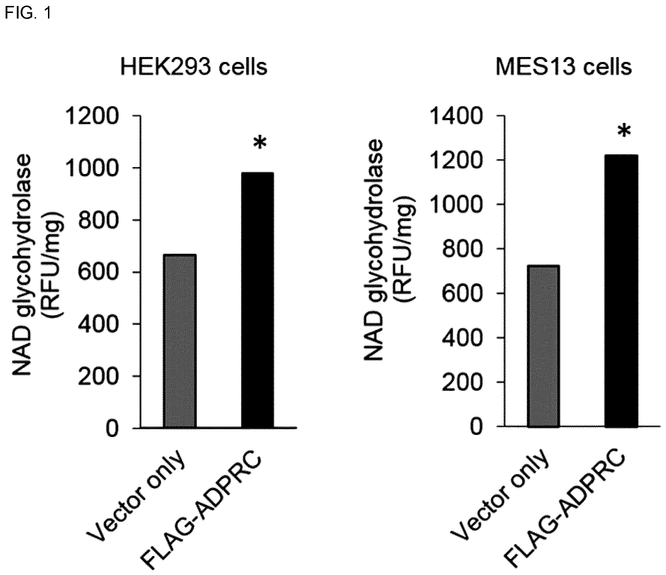

FIG. 1 shows a result of measuring the NAD glycohydrolase (NADase) activity of new ADPRC in HEK293 cells and MES13 cells.

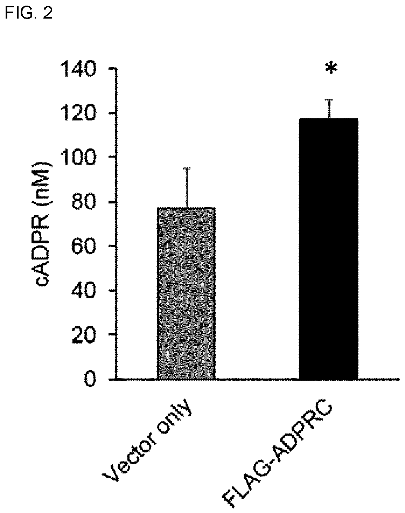

FIG. 2 shows a result of evaluating the cADPR synthesis capability of purified FLAG-ADPRC.

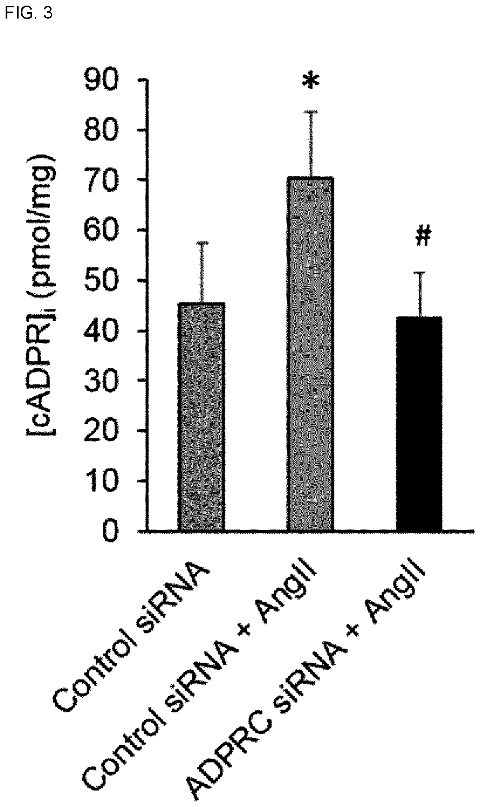

FIG. 3 shows a result of measuring the intracellular cADPR production by new ADPRC when MES13 cells are stimulated with angiotensin II.
FIG. 4 shows a result of comparing the interspecies sequence homology of ADP-ribosyl cyclase of the present disclosure (human (96%), rat (96%), dog (90%), pig (88%), rabbit (89%), sheep (90%), chicken (70%), cattle (78%), chimpanzee (93%), horse (91%), frog (63%), goat (90%), turkey (62%), guinea pig (91%), *Echinococcus granulosus* (27%), *Schistosoma haematobium* (22%), *Trichinella spiralis* (22%), *Drosophila* (19%) and zebrafish (56%) as compared to mouse).

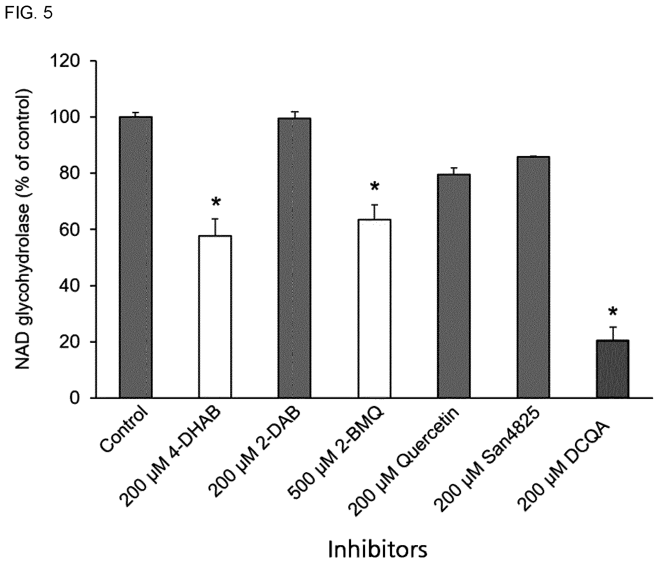

FIG. 5 shows a result of measuring the effect of new inhibitors inhibiting the NAD glycohydrolase (NADase) activity of new ADPRC.

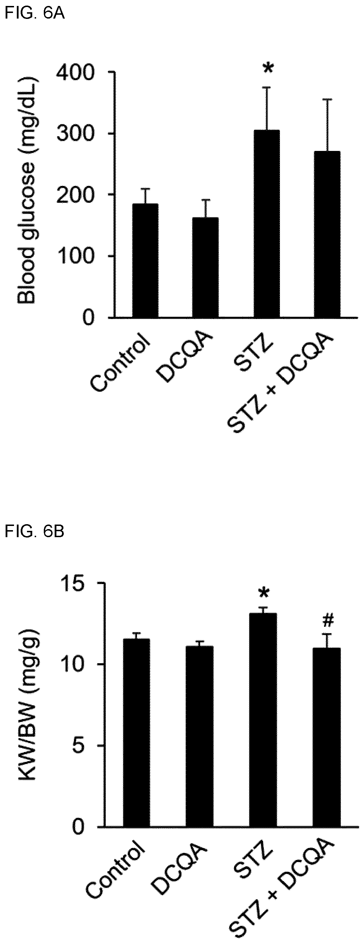

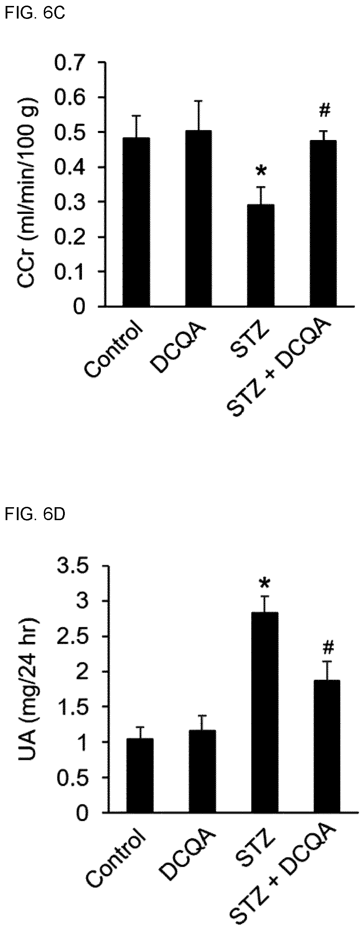

FIG. 6 shows a result of measuring the effect of dicaffeolylquinic acid (DCQA) on the blood glucose (FIG. 6A), kidney-to-body weight ratio (FIG. 6B), creatinine clearance rate (FIG. 6C) and urine albumin level (FIG. 6D) of a diabetic renal disease mouse model.

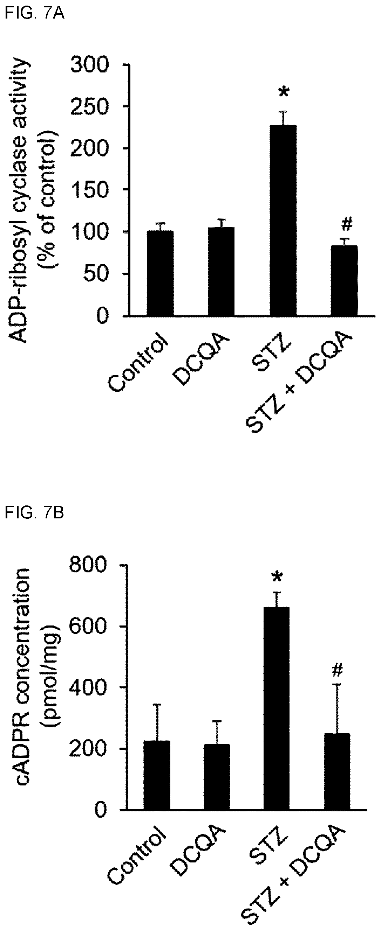

FIG. 7 shows a result of measuring the effect of DCQA on the ADPRC activity (FIG. 7A) and cADPR concentration (FIG. 7B) in the kidney tissue of a diabetic renal disease mouse model.

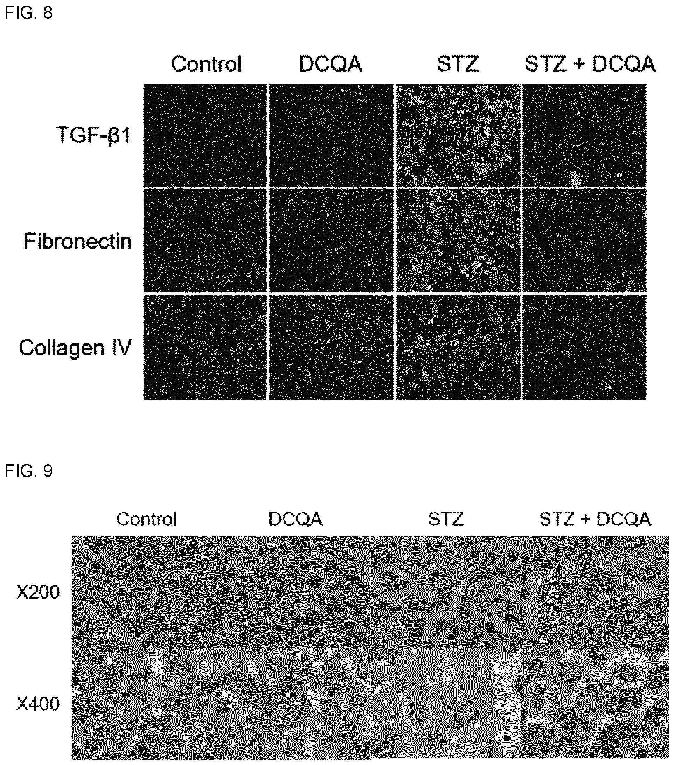

FIG. 8 shows a result of measuring the effect of DCQA on the change in expression of TGF-β1, fibronectin and collagen IV in the kidney tissue of a diabetic renal disease mouse model.
FIG. 9 shows a result of observing the histopathological change in the kidney tissue of a diabetic renal disease mouse model by hematoxylin and eosin (H&E) staining.

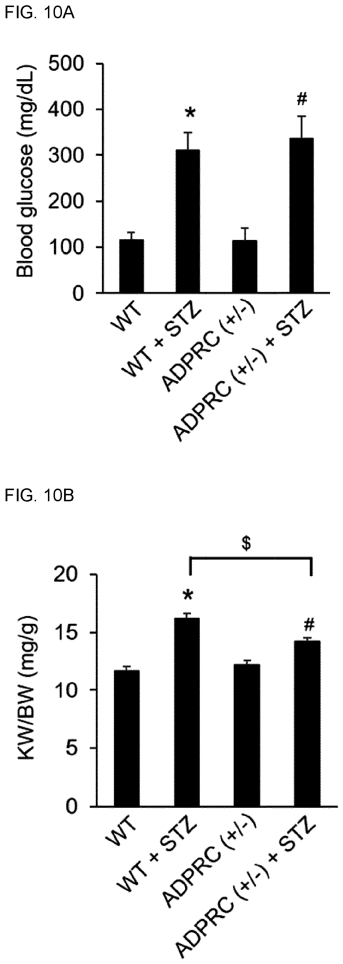

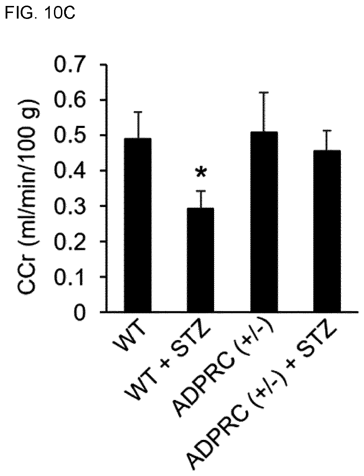

FIG. 10 shows a result of measuring the blood glucose (FIG. 10A), kidney-to-body weight ratio (FIG. 10B) and creatinine clearance rate (FIG. 10C) in normal mouse and a diabetic renal disease model of ADPRC hetero (ADPRC (+/-)) mouse.

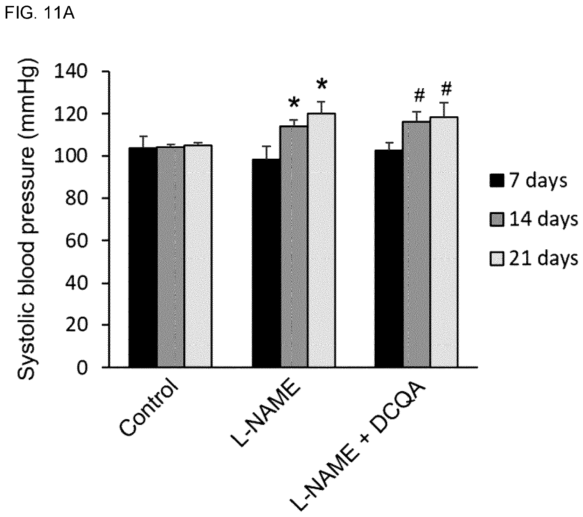

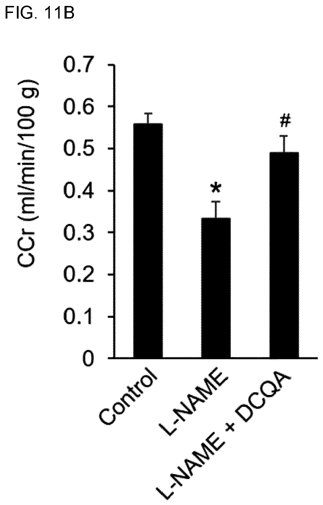

FIG. 11 shows a result of measuring the effect of DCQA on the blood pressure (FIG. 11A) and creatinine clearance rate (FIG. 11B) in normal mouse and a hypertension mouse model.

### [Best Mode]

Hereinafter, the present disclosure is described in more detail through examples. These examples are only for illustrating the present disclosure more specifically and it will be obvious to those having ordinary knowledge in the art that the scope of the present disclosure is not limited by the examples.

### Examples

### Example 1. NAD glycohydrolase (NADase) activity of new ADPRC

In order to investigate the ADP-ribosyl cyclase (ADPRC) activity of SEQ ID NO 1, a FLAG-ADPRC plasmid was prepared by ligating a cDNA sequence encoding ADPRC into a FLAG-CMV-2 vector and the overexpression of new ADPRC was induced by treating HEK293 cells or MES13 cells using a transfection reagent. The overexpressed new ADPRC was lysed with a lysis buffer. 45 µL of the lysed sample was treated with 5 µL of 2 mM ε-NAD (nicotinamide 1,N6-ethenoadenine dinucleotide) and incubated at 37 °C for 1 hour. Then, the enzyme-substrate reaction was stopped by treating with 50 µL of 10% trichloroacetic acid. After centrifuging for 10 minutes and adding 80 µL of the supernatant to 720 µL of 0.1 M sodium phosphate buffer, absorbance was measured at 297 nm (excitation) and 410 nm (emission) with a fluorescence spectrometer. The result is shown in FIG. 1.

### Example 2. Evaluation of cADPR synthesis capability of new ADPRC

The cADPR synthesis capability of ADPRC was evaluated by the method reported by Graeff R et al. [Graeff R, Lee HC. Biochem. J. 361: 379-384, 2002].

Specifically, the FLAG-ADPRC plasmid was overexpressed in HEK293 cells using a transfection reagent and then lysed with a lysis buffer. The overexpressed FLAG-ADPRC was purified from the lysed sample using a FLAG-agarose column. The purified sample was treated with 100 µM β-NAD and incubated at 37 °C for 1 hour. Then, after extracting cADPR by treating with trichloroacetic acid to a final concentration of 0.6 M, 0.1 mL of the extract or 0.1 mL of a standard cADPR solution was reacted at room temperature for 30 minutes after adding 50 µL of a mixture solution of ADPR cyclase (0.3 µg/mL), nicotinamide (30 mM) and sodium phosphate (100 mM).

After adding ethanol (2%), alcohol dehydrogenase (100 µg/mL), resazurin (20 µM), diaphorase (10 µg/mL), FMN (10 µM), nicotinamide (10 mM), bovine serum albumin (BSA, 0.1 mg/mL) and sodium phosphate (100 mM) to the mixture solution, reaction was conducted for 2-4 hours. Then, absorbance was measured between 544 nm and 590 nm using a fluorescence spectrophotometer. The result is shown in FIG. 2.

### Example 3. Change in intracellular cADPR concentration in MES13 cells by ADPRC

The change in intracellular cADPR concentration by ADPRC was investigated by the method reported by Graeff R et al. [Graeff R, Lee HC. Biochem. J. 361: 379-384, 2002].

Specifically, the expression of the new ADPRC in MES13 cells was inhibited by using a small interfering RNA (SEQ ID NO 22) as a transfection reagent. After treating the ADPRC expression-inhibited cells with 150 nM angiotensin II for 60 seconds and then extracting cADPR with 0.6 M trichloroacetic acid, 0.1 mL of the extract or 0.1 mL of a standard cADPR solution was reacted at room temperature for 30 minutes after adding 50 µL of a mixture solution of ADPR cyclase (0.3 µg/mL), nicotinamide (30 mM) and sodium phosphate (100 mM).

After adding ethanol (2%), alcohol dehydrogenase (100 µg/mL), resazurin (20 µM), diaphorase (10 µg/mL), FMN (10 µM), nicotinamide (10 mM), bovine serum albumin (BSA, 0.1 mg/mL) and sodium phosphate (100 mM) to the mixture solution, reaction was conducted for 2-4 hours. Then, absorbance was measured between 544 nm and 590 nm using a fluorescence spectrophotometer. The result is shown in FIG. 3.

### Example 4. Effect of new inhibitor inhibiting NAD glycohydrolase (NADase) activity of new ADPRC

In order to find an inhibitor which inhibits the activation of new ADP-ribosyl cyclase (ADPRC) of SEQ ID NO 1, 5 µL of each of 4,4'-dihydroxyazobenzene (4-DHAB, TCI (Japan)), 2,2'-dihydroxyazobenzene (2-DAB, Sigma-Aldrich (USA)), 2-(3,4-dihydroxyphenyl)-3,5,7-trihydroxychromen-4-one (Quercetin, Sigma-Aldrich (USA)) and San4825 (synthesized by Kannt A et al. [Kannt A, Sicka K, Kroll K, Kadereit D, Gogelein H. Naunyn. Schmiedebergs. Arch. Pharmacol. 385: 717-727, 2012], China) and 2-(1,3-benzoxazol-2-ylamino)-1-methylquinazoline-4(1H)-one (2-BMQ) and 1,4-dicaffeoylquinic acid (1,4-DCQA, Biopurify (China), hereinafter DCQA), which were newly found through screening, was reacted with 40 µL of new ADPRC in iced water for 15 minutes. Then, reaction was conducted at 37 °C for 1 hour after treating with 5 µL of 2 mM ε-NAD (nicotinamide 1,N6-ethenoadenine dinucleotide). Then, the enzyme-substrate reaction was stopped by treating with 50 µL of 10% trichloroacetic acid. After centrifuging for 10 minutes and adding 80 µL of the supernatant to 720 µL of 0.1 M sodium phosphate buffer, absorbance was measured at 297 nm (excitation) and 410 nm (emission) using a fluorescence spectrometer. The result is shown in FIG. 5. As shown in FIG. 5, the NAD glycohydrolase (NADase) activity was inhibited by 42.40% for 4-DHAB, 36.49% for 2-BMQ and even 79.64% for DCQA as compared to the control group.

### Example 5. Effect of DCQA on blood glucose, kidney-to-body weight ratio, creatinine clearance rate and urine albumin level in renal disease mouse model

The blood glucose, kidney-to-body weight ratio, creatinine clearance rate and urine albumin level in a renal disease mouse model were measured by the method reported by Kim SY et al. [Kim SY, Park KH, Gul R, Jang KY, Kim UH. Am. J. Physiol. Renal Physiol. 296: F291-F297, 2009]

Specifically, 0.2 mL of 50 mM citrate buffer (pH 4.8) (control group and DCQA group) or 200 µL of 5 mg/mL streptozotocin (STZ, Sigma-Aldrich (USA)) dissolved in 50 mM citrate buffer (STZ group and STZ+DCQA group) was intraperitoneally injected to C57BL/6J mice. Blood glucose was measured from day 2 after the STZ injection and the mice whose blood glucose level was 300 mg/dL were divided into an STZ group and an STZ+DCQA group. To the mice that showed increased blood glucose level, DCQA which showed the highest inhibitory effect against novel ADPRC in Example 5 was intraperitoneally administered every day (100 µL) for 6 weeks after dissolving in dimethyl sulfoxide to 9 mg/mL and diluting with saline to 9 µg/mL. On the last day, the mouse was put in a metabolic cage and urine was collected for 24 hours.

Then, the body weight and blood glucose of the mouse were measured (FIG. 6A). In addition, in order to investigate the possibility as a therapeutic agent for a renal disease, the mouse was sacrificed and serum and kidney were taken. After measuring kidney-to-body weight ratio (FIG. 6B), some of the kidney was fixed in 10% formalin for fluorescent staining and H&E staining, and the remainder was subjected to ADPR cyclase activity and cADPR concentration measurement. Creatinine clearance rate was calculated by measuring the creatinine levels in urine and serum using a creatinine assay kit (Bioassay Systems, USA) (FIG. 6C), and urine albumin level was measured using an albumin assay kit (Bioassay Systems, USA) (FIG. 6D). The result is shown in FIGS. 6A to 6D.

As can be seen from FIGS. 6A to 6D, although the STZ+DCQA group did not show decreased in blood glucose as compared to the control group (STZ) (FIG. 6A), the possibility as a candidate for a therapeutic agent for a renal disease such as chronic renal failure or diabetic nephropathy was confirmed from the significant decrease in kidney to body weight (FIG. 6B), significant increase in creatinine clearance rate (FIG. 6C) and significant decrease in the urine albumin level (FIG. 6D).

### Example 6. Effect of DCQA on ADPRC activity and cADPR concentration in kidney tissue of renal disease mouse model

The ADPRC activity and cADPR concentration in the kidney tissue of a renal disease mouse model were measured by the method reported by Kim SY et al. [Kim SY, Park KH, Gul R, Jang KY, Kim UH. Am. J. Physiol. Renal Physiol. 296: F291-F297, 2009]

Specifically, 0.2 mL of 50 mM citrate buffer (pH 4.8) (control group and DCQA group) or 200 µL of 5 mg/mL streptozotocin (STZ, Sigma-Aldrich (USA)) dissolved in 50 mM citrate buffer (STZ group and STZ+DCQA group) was intraperitoneally injected to C57BL/6J mice. Blood glucose was measured from day 2 after the STZ injection and the mice whose blood glucose level was 300 mg/dL were divided into an STZ group and an STZ+DCQA group. To the mice that showed increased blood glucose level, DCQA which showed the highest inhibitory effect against novel ADPRC in Example 5 was intraperitoneally administered every day (100 µL) for 6 weeks after dissolving in dimethyl sulfoxide to 9 mg/mL and diluting with saline to 9 µg/mL. In order to investigate the possibility as a therapeutic agent for a renal disease, the mouse was sacrificed and serum and kidney were taken.

After lysing some of the kidney tissue with a lysis buffer, 45 µL of the lysed sample was treated with 5 µL of 2 mM NGD (nicotinamide guanine dinucleotide) and reacted at 37 °C for 1 hour. Then, the enzyme-substrate reaction was stopped by treating with 50 µL of 10% trichloroacetic acid. After centrifuging for 10 minutes and adding 80 µL of the supernatant to 720 µL of a 0.1 M sodium phosphate buffer, absorbance was measured at 297 nm (excitation) and 410 nm (emission) with a fluorescence spectrometer (Hitachi, Japan).

In addition, after extracting cADPR by treating some of the kidney tissue taken from each group with 0.2 mL of 0.6 M trichloroacetic acid, 0.1 mL of the extract or 0.1 mL of a standard cADPR solution was reacted at room temperature for 30 minutes after adding 50 µL of a mixture solution of ADPR cyclase (0.3 µg/mL), nicotinamide (30 mM) and sodium phosphate (100 mM). The mixture solution was reacted for 2-4 hours after adding ethanol (2%), alcohol dehydrogenase (100 µg/mL), resazurin (20 µM), diaphorase (10 µg/mL), FMN (10 µM), nicotinamide (10 mM), bovine serum albumin (BSA, 0.1 mg/mL) and sodium phosphate (100 mM). Then, absorbance was measured between 544 nm and 590 nm using a fluorescence spectrophotometer. The result is shown in FIG. 7.

As shown in FIG. 7A, the DCQA of the present disclosure decreased the ADP-ribosyl cyclase activity increased by STZ to the level of the control group. And, as shown in FIG. 7B, the DCQA of the present disclosure decreased the cADPR concentration increased by STZ to the level of the control group. Therefore, it was confirmed that DCQA has the ability to decrease ADPRC activity and cADPR concentration, which are increased due to failure or loss of kidney function associated with a renal disease.

### Example 7. Effect of 1,4-DCQA on change in expression of TGF-β1, fibronectin and collagen IV in kidney tissue of renal disease mouse model

The change in the expression of TGF-β1, fibronectin and collagen IV in the kidney tissue of a renal disease mouse model was measured by the method reported by Kim SY et al. [Kim SY, Park KH, Gul R, Jang KY, Kim UH. Am. J. Physiol. Renal Physiol. 296: F291-F297, 2009].

Specifically, 0.2 mL of 50 mM citrate buffer (pH 4.8) (control group and DCQA group) or 200 µL of 5 mg/mL streptozotocin (STZ, Sigma-Aldrich (USA)) dissolved in 50 mM citrate buffer (STZ group and STZ+DCQA group) was intraperitoneally injected to C57BL/6J mice. Blood glucose was measured from day 2 after the STZ injection and the mice whose blood glucose level was 300 mg/dL were divided into an STZ group and an STZ+DCQA group. To the mice that showed increased blood glucose level, DCQA which showed the highest inhibitory effect against novel ADPRC in Example 5 was intraperitoneally administered every day (100 µL) for 6 weeks after dissolving in dimethyl sulfoxide to 9 mg/mL and diluting with saline to 9 µg/mL. In order to investigate the possibility as a therapeutic agent for a renal disease, the mouse was sacrificed and serum and kidney were taken. Some of the extracted kidney was fixed in 10% formalin. The kidney tissue fixed in 10% formalin was cut into kidney tissue sections on a slide glass using a cryostat microtome. The kidney tissue sections were washed with a TTBS (Tris-buffered saline (TBS) with 0.1% Tween 20) buffer and then incubated with a TTBS buffer containing 1% bovine serum albumin (BSA) for 1 hour. The tissues were reacted with primary antibodies (TGF-β1 (Santa Cruz, USA), fibronectin (Santa Cruz, USA) and collagen IV (Abcam, UK)) diluted in a TTBS buffer containing 1% bovine serum albumin (BSA) to 1:200 at 4 °C for 12 hours or longer. After washing the tissues that reacted with the primary antibodies 3 times with a TTBS buffer, they were reacted with FITC-labeled secondary antibodies diluted in a TTBS buffer to 1:200 in the dark at room temperature for 1 hour. Then, after washing 3 times with a TTBS buffer, a cover glass was attached using a mounting solution. The stained kidney tissue was observed using a fluorescence microscope (Carl Zeiss, Germany) for observing green fluorescence. The result is shown in FIG. 8.

As shown in FIG. 8, the DCQA of the present disclosure decreased the expression level of TGF-β1, fibronectin and collagen IV increased by STZ to the level of the control group. Therefore, it was confirmed that DCQA has the ability to decrease the expression level of TGF-β1, fibronectin and collagen IV, which are increased due to failure or loss of kidney function associated with a renal disease.

### Example 8. Investigation of histopathological change in kidney tissue of renal disease mouse model through hematoxylin and eosin (H&E) staining

The histopathological change in the kidney tissue of a renal disease mouse model was measured by the method reported by Shu B et al. [Shu B, Feng Y, Gui Y, Lu Q, Wei W, Xue X, Sun X, He W, Yang J, Dai C. Cell. Signal. 42:249-258, 2018]

Specifically, 0.2 mL of 50 mM citrate buffer (pH 4.8) (control group and DCQA group) or 200 µL of 5 mg/mL streptozotocin (STZ, Sigma-Aldrich (USA)) dissolved in 50 mM citrate buffer (STZ group and STZ+DCQA group) was intraperitoneally injected to C57BL/6J mice. Blood glucose was measured from day 2 after the STZ injection and the mice whose blood glucose level was 300 mg/dL were divided into an STZ group and an STZ+DCQA group. To the mice that showed increased blood glucose level, DCQA which showed the highest inhibitory effect against novel ADPRC in Example 5 was intraperitoneally administered every day (100 µL) for 6 weeks after dissolving in dimethyl sulfoxide to 9 mg/mL and diluting with saline to 9 µg/mL. In order to investigate the possibility as a therapeutic agent for a renal disease, the mouse was sacrificed and serum and kidney were taken. Some of the extracted kidney was fixed in 10% formalin. The kidney tissue fixed in 10% formalin was cut into kidney tissue sections on a slide glass using a cryostat microtome. The kidney tissue sections were washed with running water for 5 minutes and then stained with hematoxylin for 5 minutes. After the staining, the tissue was washed with running water for 5 minutes. Then, the tissue was immersed in 157 mM hydrochloric acid 2 times and taken out quickly. Then, the tissue was immersed once in 0.25% ammonia water and taken out quickly. After washing again with running water for 5 minutes and staining with eosin for about 30 seconds, the tissue was reacted with 70% ethanol for 30 seconds, with 80% ethanol for 30 seconds, with 90% ethanol for 30 seconds, with 100% ethanol for 30 seconds, again with 100% ethanol for 30 seconds, and then again with 100% ethanol for 30 seconds. Finally, after reacting with xylene for 5 minutes and again with xylene for 5 minutes or longer, a cover glass was attached using a mounting solution. The stained kidney tissue was observed with an optical microscope (Lieca, Germany). The result is shown in FIG. 9.

As shown in FIG. 9, the DCQA of the present disclosure recovered the formation of glomerulus hypertrophy, infiltration of inflammatory cells and formation of transitional epithelial cells increased by STZ to a level similar to that of the control group. Therefore, it was confirmed that DCQA has the ability to recover the histopathological changes of the kidney caused by failure or loss of kidney function associated with a renal disease.

### Example 9. Comparison of blood glucose, kidney-to-body weight ratio and creatinine clearance rate between normal mouse and ADPRC hetero (ADPRC (+/-)) mouse renal disease model

The blood glucose, kidney-to-body weight ratio and creatinine clearance rate of a renal disease mouse model were measured by the method reported by Kim SY et al. [Kim SY, Park KH, Gul R, Jang KY, Kim UH. Am. J. Physiol. Renal Physiol. 296: F291-F297, 2009]

Specifically, 200 µL of streptozotocin (STZ) dissolved in a 50 mM citrate buffer (pH 4.8) to 5 mg/mL was intraperitoneally injected to 129S1/SvlmJ mouse (wild type, WT) and ADPRC hetero knockout (ADPRC(+/-)) mouse acquired from The Jackson Laboratory (USA). Blood glucose was measured from day 2 after the injection and the mice whose blood glucose level was 300 mg/dL were used. 6 weeks later, the mouse was put in a metabolic cage and urine was collected for 24 hours.

Then, the body weight and blood glucose of the mouse were measured (FIG. 10A). The mouse was sacrificed and serum and kidney were taken. Then, kidney-to-body weight ratio was measured using the kidney (FIG. 10B). Creatinine clearance rate was calculated by measuring the creatinine levels in urine and serum using a creatinine assay kit (Bioassay Systems, USA) (FIG. 10C). The result is shown in FIGS. 10A to 10C.

As shown in FIGS. 10A to 10C, although blood glucose was not decreased in the (ADPRC(+/-) group as compared to the control group (FIG. 10A), significant decrease in kidney to body weight was observed in the ADPRC(+/-)+STZ group of the present disclosure as compared to the WT+STZ group (FIG. 10B). In addition, the decrease in creatinine clearance rate caused by STZ was not observed in the ADPRC(+/-)+STZ group (FIG. 10C), which confirms the importance of the new ADPRC in a renal disease such as chronic renal failure or diabetic nephropathy.

### Example 10. Effect of DCQA on blood pressure and creatinine clearance rate in normal mouse and hypertension mouse model

The blood pressure and creatinine clearance rate in a hypertension mouse model were measured by the method reported by Allagnat et al. [Allagnat F, Haefliger JA, Lambelet M, Longchamp A, Berard X, Mazzolai L, Corpataux JM, Deglise S. Eur. J. Vasc. Endovasc. Surg. 51: 733-742, 2016] and Kim SY et al. [Kim SY, Park KH, Gul R, Jang KY, Kim UH. Am. J. Physiol. Renal Physiol. 296: F291-F297, 2009]

Specifically, 0.2 mL of 8 mg of L-NAME (Nw-nitro-L-arginine-methyl-ester, Sigma-Aldrich, USA) dissolved in 1 mL of saline was orally administered to C57BL/6J mouse every day. On days 7 and 14 of the oral administration, blood pressure was measured at the tail of the mouse. Then, DCQA which showed the highest inhibitory effect against novel ADPRC in Example 5 was intraperitoneally administered every day (100 µL) for 7 days after dissolving in dimethyl sulfoxide to 9 mg/mL and diluting with saline to 9 µg/mL and L-NAME was administered orally. On day 6 after the DCQA treatment, the mouse was put in a metabolic cage and urine was collected for 24 hours.

After measuring the blood pressure of the mouse (FIG. 11A), the mouse was sacrificed and serum was obtained.

Creatinine clearance rate was calculated by measuring the creatinine levels in urine and serum using a creatinine assay kit (Bioassay Systems, USA) (FIG. 11B). The result is shown in FIGS. 11A and 11B.

As shown in FIG. 11A, DCQA had no effect of lowering blood pressure in the hypertension model. However, the possibility of DCQA as a therapeutic agent for a renal disease such as hypertensive nephropathy was confirmed through the increase in creatinine clearance rate by DCQA (FIG. 11B).

### <References>

1. Berridge MJ, Bootman MD, Roderick HL. Calcium signaling: dynamics, homeostasis and remodeling. Nat Rev Mol Cell Biol. 4: 517-529, 2003.
2. Lee HC. Structure and enzymatic functions of human CD38. Mol Med. 12: 317-323, 2006.
3. Resnick LM. Ionic basis of hypertension, insulin resistance, vascular disease, and related disorders. The mechanism of "Syndrome X". Am. J. Hypertens. 6:123S-134S, 1993.
4. Cowley AW Jr. Long-term control of arterial blood pressure. Physiol. Rev. 72: 231-300, 1992.
5. Kim BJ, Park KH, Yim CY, Takasawa S, Okamoto H, Im MJ, Kim UH. Generation of nicotinic acid adenine dinucleotide phosphate and cyclic ADP-ribose by glucagon-like peptide-1 evokes Ca2+ signal that is essential for insulin secretion in mouse pancreatic islets. Diabetes. 57: 868-878, 2008.
6. Gul R, Park JH, Kim SY, Jang KY, Chea JK, Ko JK, Kim UH. Inhibition of ADP-ribosyl cyclase attenuates angiotensin II-induced cardiac hypertrophy. Cardiovasc. Res. 81: 582-591, 2009.
7. Kim SY, Gul R, Rah SY, Kim SH, Park SK, Im MJ, Kwon HJ, Kim UH. Molecular mechanism of ADP-ribosyl cyclase activation in angiotensin II signaling in murine mesangial cells. Am. J. Physiol. Renal. Physiol. 294: F989-F989, 2008.
8. Partida-Sanchez S, Cockayne DA, Monard S, Jacobson EL, Oppenheimer N, Garvy B, Kusser K, Goodrich S, Howard M, Harmsen A, Randall TD, Lund FE. Cyclic ADP-ribose production by CD38 regulates intracellular calcium release, extracellular calcium influx and chemotaxis in neutrophils and is required for bacterial clearance in vivo. Nat Med 7: 1209-1216, 2001.
9. Graeff R, Lee HC. A novel cycling assay for cellular cADP-ribose with nanomolar sensitivity. Biochem. J. 361: 379-384, 2002.
10. Kim SY, Park KH, Gul R, Jang KY, Kim UH. Role of kidney ADP-ribosyl cyclase in diabetic nephropathy. Am. J. Physiol. Renal Physiol. 296: F291-F297, 2009.
11. Shu B, Feng Y, Gui Y, Lu Q, Wei W, Xue X, Sun X, He W, Yang J, Dai C. Blockade of CD38 diminishes lipopolysaccharide-induced macrophage classical activation and acute kidney injury involving NF-κB signaling suppression. Cell. Signal. 42: 249-258, 2018.
12. Allagnat F, Haefliger JA, Lambelet M, Longchamp A, Berard X, Mazzolai L, Corpataux JM, Deglise S. Nitric oxide deficit drives intimal hyperplasia in mouse models of hypertension. Eur. J. Vasc. Endovasc. Surg. 51: 733-742, 2016.
13. Kannt A, Sicka K, Kroll K, Kadereit D, Gogelein H. Naunyn. Schmiedebergs. Arch. Pharmacol. 385: 717-727, 2012.

Although the specific exemplary embodiments of the present disclosure have been described in detail, it will be obvious to those having ordinary knowledge in the art that they are merely preferred exemplary embodiments and the scope of the present disclosure is not limited by them. It is to be understood that the substantial scope of the present disclosure is defined by the appended claims and their equivalents.

## Claims

1. An ADP-ribosyl cyclase (ADPRC) comprising an amino acid sequence of SEQ ID NO 1 or a naturally occurring variant thereof.

2. The ADP-ribosyl cyclase or naturally occurring variant thereof according to claim 1, wherein the naturally occurring variant of ADP-ribosyl cyclase is a naturally occurring variant selected from a group consisting of an interspecies variant, a species homolog, an isoform, an allelic variant, a conformational variant, a splice variant and a point mutation variant.

3. The ADP-ribosyl cyclase or naturally occurring variant thereof according to claim 1, wherein the naturally occurring variant of ADP-ribosyl cyclase originates from an organism selected from a group consisting of mammals, birds, reptiles, amphibians and fish.

4. The ADP-ribosyl cyclase or naturally occurring variant thereof according to claim 1, wherein the naturally occurring variant of ADP-ribosyl cyclase is an ADP-ribosyl cyclase comprising an amino acid sequence selected from a group consisting of SEQ ID NOS 2-21.

5. The ADP-ribosyl cyclase or naturally occurring variant thereof according to claim 1, wherein the ADP-ribosyl cyclase or variant thereof converts NAD⁺ to cyclic ADP-ribose (cADPR).

6. A nucleic acid molecule encoding the ADP-ribosyl cyclase or naturally occurring variant thereof according to claim 1.

7. A vector comprising the nucleic acid molecule according to claim 6.

8. A host cell comprising the vector according to claim 7.

9. A catalyst composition which converts NAD⁺ to cyclic ADP-ribose (cADPR), comprising the ADP-ribosyl cyclase or naturally occurring variant thereof according to claim 1, the nucleic acid molecule according to claim 6 or the vector according to claim 7.

10. A pharmaceutical composition for preventing or treating an ADP-ribosyl cyclase-mediated disease, comprising an inhibitor against the expression or activation of an ADP-ribosyl cyclase comprising an amino acid sequence of SEQ ID NO 1 or a naturally occurring variant thereof as an active ingredient.

11. The pharmaceutical composition according to claim 10, wherein the inhibitor against the expression of an ADP-ribosyl cyclase or a naturally occurring variant thereof is selected from a group consisting of an antisense oligonucleotide, a siRNA, a shRNA, a miRNA, a ribozyme, a DNAzyme and a PNA (protein nucleic acid).

12. The pharmaceutical composition according to claim 11, wherein the siRNA comprises a nucleotide sequence of SEQ ID NO 22.

13. The pharmaceutical composition according to claim 10, wherein the inhibitor against the activation of the ADP-ribosyl cyclase or naturally occurring variant thereof is selected from a group consisting of a compound, a peptide, a peptide mimetic, an aptamer and an antibody.

14. The pharmaceutical composition according to claim 13, wherein the compound is selected from a group consisting of 4,4'-dihydroxyazobenzene, 2-(1,3-benzoxazol-2-ylamino)-1-methylquinazolin-4(1H)-one and dicaffeoylquinic acid.

15. The pharmaceutical composition according to claim 10, wherein the ADP-ribosyl cyclase-mediated disease is a renal disease.

16. The pharmaceutical composition according to claim 15, wherein the renal disease is renal failure, nephropathy, nephritis, renal fibrosis or nephrosclerosis.

17. The pharmaceutical composition according to claim 16, wherein the renal failure is chronic renal failure, acute renal failure or mild renal failure before dialysis.

18. The pharmaceutical composition according to claim 16, wherein the nephropathy is nephropathy syndrome, lipoid nephropathy, diabetic nephropathy, immunoglobulin A (IgA) nephropathy, analgesic nephropathy or hypertensive nephropathy.

19. A food composition for preventing or alleviating an ADP-ribosyl cyclase-mediated disease, comprising an inhibitor against the expression or activation of an ADP-ribosyl cyclase comprising an amino acid sequence of SEQ ID NO 1 or a naturally occurring variant thereof as an active ingredient.

20. A non-human animal model wherein a hetero-type gene of the ADP-ribosyl cyclase (ADPRC) or the naturally occurring variant thereof according to claim 1 is deleted.

21. A method for identifying an ADP-ribosyl cyclase-mediated disease, comprising:
(a) a step of inducing a specific disease in the animal model according to claim 20 and a wild-type animal model; and
(b) a step of identifying the difference between the animal models.

22. A method for providing information for diagnosis of an ADP-ribosyl cyclase-mediated disease, comprising:
1) a step of measuring the expression or activation level of an ADP-ribosyl cyclase comprising an amino acid sequence of SEQ ID NO 1 or a naturally occurring variant thereof in a sample isolated from a subject; and
2) a step of determining a risk of the ADP-ribosyl cyclase-mediated disease of the subject by comparing the expression or activation level of the ADP-ribosyl cyclase or naturally occurring variant thereof in the step 1) with a normal control group.

23. A composition for diagnosis of an ADP-ribosyl cyclase-mediated disease, comprising an agent for measuring the gene expression level or protein level of an ADP-ribosyl cyclase comprising an amino acid sequence of SEQ ID NO 1 or a naturally occurring variant thereof.

24. A kit for diagnosis of an ADP-ribosyl cyclase-mediated disease, comprising an agent for measuring the gene expression level or protein level of an ADP-ribosyl cyclase comprising an amino acid sequence of SEQ ID NO 1 or a naturally occurring variant thereof.

25. A method for screening a substance for preventing or treating an ADP-ribosyl cyclase-mediated disease, comprising:
1) a step of treating a cell expressing an ADP-ribosyl cyclase comprising an amino acid sequence of SEQ ID NO 1 or a naturally occurring variant thereof with a test substance;
2) a step of measuring the gene expression level or protein level of the ADP-ribosyl cyclase or naturally occurring variant thereof as a result of treating with the test substance; and
3) a step of screening the test substance as a substance for preventing or treating an ADP-ribosyl cyclase-mediated disease if the gene expression level or protein level is decreased as compared to a control group not treated with the test substance.


<110>    INDUSTRIAL COOPERATION FOUNDATION, CHONBUK NATIONAL UNIVERSITY

<120>    Novel ADP-ribosyl Cyclases and Their Inhibitors

<130>    PCT696

<150>    KR 10-2019-0076979
<151>    2019-06-27

<150>    KR 10-2020-0076635
<151>    2020-06-23

<160>    22

<170>    KoPatentIn 3.0

<210>    1
<211>    304
<212>    PRT
<213>    Artificial Sequence

<220>
<223>    Mouse ADP-ribosyl Cyclase


<400>    1
Met Ala Arg Gly Lys Ala Lys Glu Glu Gly Ser Trp Lys Lys Phe Ile
  1               5                  10                  15

Trp Asn Ser Glu Lys Lys Glu Phe Leu Gly Arg Thr Gly Gly Ser Trp
             20                  25                  30

Phe Lys Ile Leu Leu Phe Tyr Val Ile Phe Tyr Gly Cys Leu Ala Gly
         35                  40                  45

Ile Phe Ile Gly Thr Ile Gln Val Met Leu Leu Thr Ile Ser Glu Leu
     50                  55                  60

Lys Pro Thr Tyr Gln Asp Arg Val Ala Pro Pro Gly Leu Thr Gln Ile
 65                  70                  75                  80

Pro Gln Ile Gln Lys Thr Glu Ile Ser Phe Arg Pro Asn Asp Pro Lys
                 85                  90                  95

Ser Tyr Glu Ala Tyr Val Leu Asn Ile Ile Arg Phe Leu Glu Lys Tyr
            100                 105                 110

Lys Asp Ser Ala Gln Lys Asp Asp Met Ile Phe Glu Asp Cys Gly Asn
        115                 120                 125

Val Pro Ser Glu Pro Lys Glu Arg Gly Asp Ile Asn His Glu Arg Gly
    130                 135                 140

Glu Arg Lys Val Cys Arg Phe Lys Leu Asp Trp Leu Gly Asn Cys Ser
145                 150                 155                 160

Gly Leu Asn Asp Asp Ser Tyr Gly Tyr Arg Glu Gly Lys Pro Cys Ile
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Leu Met Met Lys Tyr Asn Pro Asn

195                 200                 205

Val Leu Pro Val Gln Cys Thr Gly Lys Arg Asp Glu Asp Lys Asp Lys
    210                 215                 220

Val Gly Asn Ile Glu Tyr Phe Gly Met Gly Gly Tyr Tyr Gly Phe Pro
225                 230                 235                 240

Leu Gln Tyr Tyr Pro Tyr Tyr Gly Lys Leu Leu Gln Pro Lys Tyr Leu
                245                 250                 255

Gln Pro Leu Leu Ala Val Gln Phe Thr Asn Leu Thr Val Asp Thr Glu
            260                 265                 270

Ile Arg Val Glu Cys Lys Ala Tyr Gly Glu Asn Ile Gly Tyr Ser Glu
        275                 280                 285

Lys Asp Arg Phe Gln Gly Arg Phe Asp Val Lys Ile Glu Ile Lys Ser
    290                 295                 300

<210>    2
<211>    303
<212>    PRT
<213>    Artificial Sequence

<220>
<223>    Human ADP-ribosyl Cyclase

<400>    2
Met Ala Arg Gly Lys Ala Lys Glu Glu Gly Ser Trp Lys Lys Phe Ile
  1               5                  10                  15

Trp Asn Ser Glu Lys Lys Glu Phe Leu Gly Arg Thr Gly Gly Ser Trp
             20                  25                  30

Phe Lys Ile Leu Leu Phe Tyr Val Ile Phe Tyr Gly Cys Leu Ala Gly
         35                  40                  45

Ile Phe Ile Gly Thr Ile Gln Val Met Leu Leu Thr Ile Ser Glu Phe
     50                  55                  60

Lys Pro Thr Tyr Gln Asp Arg Val Ala Pro Pro Gly Leu Thr Gln Ile
 65                  70                  75                  80

Pro Gln Ile Gln Lys Thr Glu Ile Ser Phe Arg Pro Asn Asp Pro Lys
                 85                  90                  95

Ser Tyr Glu Ala Tyr Val Leu Asn Ile Val Arg Phe Leu Glu Lys Tyr
            100                 105                 110

Lys Asp Ser Ala Gln Arg Asp Asp Met Ile Phe Glu Asp Cys Gly Asp
        115                 120                 125

Val Pro Ser Glu Pro Lys Glu Arg Gly Asp Phe Asn His Glu Arg Gly
    130                 135                 140

Glu Arg Lys Val Cys Arg Phe Lys Leu Glu Trp Leu Gly Asn Cys Ser
145                 150                 155                 160

Gly Leu Asn Asp Glu Thr Tyr Gly Tyr Lys Glu Gly Lys Pro Cys Ile
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Val Met Lys Tyr Asn Pro Asn Val
        195                 200                 205

Leu Pro Val Gln Cys Thr Gly Lys Arg Asp Glu Asp Lys Asp Lys Val
    210                 215                 220

Gly Asn Val Glu Tyr Phe Gly Leu Gly Asn Ser Pro Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Leu Ala Val Gln Phe Thr Asn Leu Thr Met Asp Thr Glu Ile
            260                 265                 270

Arg Ile Glu Cys Lys Ala Tyr Gly Glu Asn Ile Gly Tyr Ser Glu Lys
        275                 280                 285

Asp Arg Phe Gln Gly Arg Phe Asp Val Lys Ile Glu Val Lys Ser
    290                 295                 300

<210>    3
<211>    304
<212>    PRT
<213>    Artificial Sequence

<220>
<223>    Rat ADP-ribosyl Cyclase

<400>    3
Met Ala Arg Gly Lys Ala Lys Glu Glu Gly Ser Trp Lys Lys Phe Ile
1               5                   10                  15

Trp Asn Ser Glu Lys Lys Glu Phe Leu Gly Arg Thr Gly Gly Ser Trp
            20                  25                  30

Phe Lys Ile Leu Leu Phe Tyr Val Ile Phe Tyr Gly Cys Leu Ala Gly
        35                  40                  45

Ile Phe Ile Gly Thr Ile Gln Val Met Leu Leu Thr Ile Ser Glu Leu
    50                  55                  60

Lys Pro Thr Tyr Gln Asp Arg Val Ala Pro Pro Gly Leu Thr Gln Ile
65                  70                  75                  80

Pro Gln Ile Gln Lys Thr Glu Ile Ser Phe Arg Pro Asn Asp Pro Lys
                85                  90                  95

Ser Tyr Glu Ala Tyr Val Leu Asn Ile Ile Arg Phe Leu Glu Lys Tyr
            100                 105                 110

Lys Asp Ser Ala Gln Lys Asp Asp Met Ile Phe Glu Asp Cys Gly Ser
        115                 120                 125

Met Pro Ser Glu Pro Lys Glu Arg Gly Glu Phe Asn His Glu Arg Gly
    130                 135                 140

Glu Arg Lys Val Cys Arg Phe Lys Leu Asp Trp Leu Gly Asn Cys Ser
145                 150                 155                 160

Gly Leu Asn Asp Glu Ser Tyr Gly Tyr Lys Glu Gly Lys Pro Cys Ile
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Leu Thr Met Lys Tyr Asn Pro Asn
        195                 200                 205

Val Leu Pro Val Gln Cys Thr Gly Lys Arg Asp Glu Asp Lys Asp Lys
    210                 215                 220

Val Gly Asn Ile Glu Tyr Phe Gly Met Gly Gly Phe Tyr Gly Phe Pro
225                 230                 235                 240

Leu Gln Tyr Tyr Pro Tyr Tyr Gly Lys Leu Leu Gln Pro Lys Tyr Leu
                245                 250                 255

Gln Pro Leu Leu Ala Val Gln Phe Thr Asn Leu Thr Leu Asp Thr Glu
            260                 265                 270

Ile Arg Ile Glu Cys Lys Ala Tyr Gly Glu Asn Ile Gly Tyr Ser Glu
        275                 280                 285

Lys Asp Arg Phe Gln Gly Arg Phe Asp Val Lys Ile Glu Val Lys Ser
    290                 295                 300

<210>    4
<211>    303
<212>    PRT
<213>    Artificial Sequence

<220>
<223>    Dog ADP-ribosyl Cyclase

<400>    4
Met Ala Arg Gly Lys Ala Lys Glu Glu Gly Ser Trp Lys Lys Phe Ile
  1               5                  10                  15

Trp Asn Ser Glu Lys Lys Glu Phe Leu Gly Arg Thr Gly Gly Ser Trp
             20                  25                  30

Phe Lys Ile Leu Leu Phe Tyr Val Ile Phe Tyr Gly Cys Leu Ala Gly
         35                  40                  45

Ile Phe Ile Gly Thr Ile Gln Val Met Leu Leu Thr Ile Ser Glu Phe
     50                  55                  60

Lys Pro Thr Tyr Gln Asp Arg Val Ala Pro Pro Gly Leu Thr Gln Ile
 65                  70                  75                  80

Pro Gln Ile Gln Lys Thr Glu Ile Ser Phe Arg Pro Asn Asp Pro Lys

85                  90                  95

Ser Tyr Glu Glu Tyr Val Arg Asn Ile Val Arg Phe Leu Glu Lys Tyr
            100                 105                 110

Lys Asp Ser Ala Gln Lys Asp Glu Met Ile Phe Glu Asp Cys Gly Asn
        115                 120                 125

Met Pro Ser Glu Ile Lys Glu Arg Gly Glu Phe Asn Asn Glu Arg Gly
    130                 135                 140

Glu Arg Lys Val Cys Arg Phe Lys Leu Glu Trp Leu Gly Asn Cys Ser
145                 150                 155                 160

Gly Ile Asn Asp Glu Thr Tyr Gly Tyr Arg Asp Gly Lys Pro Cys Val
                165                 170                 175

Leu Ile Lys Leu Asn Arg Val Leu Gly Phe Lys Pro Lys Pro Pro Lys
            180                 185                 190

Asn Glu Ser Leu Glu Ala Tyr Pro Val Met Lys Tyr Asn Pro Tyr Val
        195                 200                 205

Leu Pro Val Gln Cys Thr Gly Lys Arg Asp Glu Asp Lys Asp Arg Ile
    210                 215                 220

Gly Asn Val Glu Tyr Phe Gly Leu Gly Gly Tyr Pro Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Leu Ala Val Gln Phe Thr Asn Leu Thr Met Asp Thr Glu Ile
            260                 265                 270

Arg Ile Glu Cys Lys Ala Tyr Gly Glu Asn Ile Gly Tyr Ser Glu Lys
        275                 280                 285

Asp Arg Phe Gln Gly Arg Phe Asp Val Lys Ile Glu Val Lys Ser
    290                 295                 300

<210>   5
<211>   303
<212>   PRT
<213>   Artificial Sequence

<220>
<223>   Pig ADP-ribosyl Cyclase

<400>   5
Met Ala Arg Gly Lys Ala Lys Glu Glu Gly Ser Trp Lys Lys Leu Met
1               5                   10                  15

Trp Asn Ser Glu Lys Lys Glu Phe Leu Gly Arg Thr Gly Gly Ser Trp
            20                  25                  30

Phe Lys Ile Leu Leu Phe Tyr Val Ile Phe Tyr Gly Cys Leu Ala Gly
        35                  40                  45

Ile Phe Ile Gly Thr Ile Gln Val Met Leu Leu Thr Ile Ser Glu Phe
    50                  55                  60

Lys Pro Thr Tyr Gln Asp Arg Val Ala Pro Pro Gly Leu Thr Gln Ile
65                  70                  75                  80

Pro Gln Ser Gln Lys Thr Glu Ile Ser Phe Arg Pro Asn Asp Pro Gln
                85                  90                  95

Ser Tyr Glu Ser Tyr Val Val Ser Ile Val Arg Phe Leu Glu Lys Tyr
            100                 105                 110

Lys Asp Leu Ala Gln Lys Asp Asp Met Ile Phe Glu Asp Cys Gly Asn
        115                 120                 125

Val Pro Ser Glu Leu Lys Glu Arg Gly Glu Tyr Asn Asn Glu Arg Gly
    130                 135                 140

Glu Arg Lys Val Cys Arg Phe Arg Leu Glu Trp Leu Gly Asn Ser Ser
145                 150                 155                 160

Gly Leu Asn Asp Glu Thr Tyr Gly Tyr Lys Asp Gly Lys Pro Cys Val
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Val Met Lys Tyr Asn Pro Tyr Val
        195                 200                 205

Leu Pro Val His Cys Thr Gly Lys Arg Asp Glu Asp Lys Glu Lys Val
    210                 215                 220

Gly Thr Met Glu Tyr Phe Gly Leu Gly Gly Tyr Pro Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Met Ala Val Gln Phe Thr Asn Leu Thr Met Asp Thr Glu Ile
            260                 265                 270

Arg Ile Glu Cys Lys Ala Tyr Gly Glu Asn Ile Gly Tyr Ser Glu Lys
        275                 280                 285

Asp Arg Phe Gln Gly Arg Phe Asp Val Lys Ile Glu Val Lys Ser
    290                 295                 300

<210> 6
<211> 303
<212> PRT
<213> Artificial Sequence

<220>
<223> Rabbit ADP-ribosyl Cyclase

<400> 6
Met Ala Arg Gly Lys Ala Lys Glu Glu Gly Ser Trp Lys Lys Phe Ile
1               5                   10                  15

Trp Asn Ser Glu Lys Lys Glu Phe Leu Gly Arg Thr Gly Gly Ser Trp
                20                  25                  30

Phe Lys Ile Leu Leu Phe Tyr Val Ile Phe Tyr Gly Cys Leu Ala Gly
            35                  40                  45

Ile Phe Ile Gly Thr Ile Gln Val Met Leu Leu Thr Ile Ser Glu Phe
    50                  55                  60

Lys Pro Thr Tyr Gln Asp Arg Val Ala Pro Pro Gly Leu Thr Gln Val
65                  70                  75                  80

Pro Gln Ile Gln Lys Thr Glu Ile Ala Phe Arg Pro Ser Asp Pro Lys
                85                  90                  95

Ser Tyr Glu Glu Tyr Val Val Asn Ile Val Arg Phe Leu Glu Lys Tyr
            100                 105                 110

Lys Asp Ser Ala Gln Lys Asp Asp Met Val Phe Glu Asp Cys Gly Asp
        115                 120                 125

Val Pro Ser Glu Pro Lys Glu Arg Gly Glu Phe Asn Asn Glu Arg Gly
    130                 135                 140

Gln Arg Lys Val Cys Arg Phe Lys Leu Asn Trp Leu Gly Asn Cys Ser
145                 150                 155                 160

Gly Ile Asp Asp Glu Thr Tyr Gly Tyr Lys Asp Gly Lys Pro Cys Ile
                165                 170                 175

Ile Ile Lys Leu Asn Arg Val Leu Gly Phe Lys Pro Lys Pro Pro Lys
            180                 185                 190

Asn Asp Ser Leu Glu Phe Ser Pro Gly Thr Lys Tyr Asn Pro Asn Val
        195                 200                 205

Leu Pro Val Gln Cys Thr Gly Lys Arg Asp Glu Asp Lys Glu Lys Val
    210                 215                 220

Gly Ser Met Glu Tyr Phe Gly Met Gly Asp Tyr Ala Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Leu Ala Val Gln Phe Thr Asn Leu Thr Met Asp Thr Glu Ile
            260                 265                 270

Arg Ile Glu Cys Lys Ala Tyr Gly Glu Asn Ile Gly Tyr Ser Glu Lys
        275                 280                 285

Asp Arg Phe Gln Gly Arg Phe Asp Val Lys Ile Glu Val Lys Ser
    290                 295                 300

<210>  7
<211>  303
<212>  PRT
<213>  Artificial Sequence

<220>
<223>    Sheep ADP-ribosyl Cyclase

<400>    7
Met Ala Arg Gly Lys Ala Lys Glu Glu Gly Ser Trp Lys Lys Phe Ile
1               5                   10                  15

Trp Asn Ser Glu Lys Lys Glu Phe Leu Gly Arg Thr Gly Gly Ser Trp
            20                  25                  30

Phe Lys Ile Leu Leu Phe Tyr Val Ile Phe Tyr Gly Cys Leu Ala Gly
        35                  40                  45

Ile Phe Ile Gly Thr Ile Gln Val Met Leu Leu Thr Ile Ser Glu Phe
    50                  55                  60

Lys Pro Thr Tyr Gln Asp Arg Val Ala Pro Pro Gly Leu Thr Gln Ile
65                  70                  75                  80

Pro Gln Ile Gln Lys Thr Glu Ile Ala Phe Arg Pro Asn Asp Pro Lys
                85                  90                  95

Ser Tyr Met Thr Tyr Val Asp Asn Ile Asp Asn Phe Leu Lys Lys Tyr
            100                 105                 110

Arg Asp Ser Ala Gln Lys Asp Asp Met Ile Phe Glu Asp Cys Gly Asn
        115                 120                 125

Val Pro Ser Glu Leu Lys Asp Arg Gly Glu Phe Asn Asn Glu Gln Gly
    130                 135                 140

Glu Arg Lys Val Cys Arg Phe Lys Leu Glu Trp Leu Gly Asn Cys Ser
145                 150                 155                 160

Gly Ile Asn Asp Glu Thr Tyr Gly Tyr Lys Glu Gly Lys Pro Cys Val
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Val Met Lys Tyr Asn Pro Tyr Val
        195                 200                 205

Leu Pro Val Gln Cys Thr Gly Lys Arg Asp Glu Asp Lys Glu Lys Val
    210                 215                 220

Gly Ser Ile Glu Tyr Phe Gly Leu Gly Gly Tyr Pro Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Leu Ala Val Gln Phe Thr Asn Leu Thr Met Asp Thr Glu Ile
            260                 265                 270

Arg Ile Glu Cys Lys Ala Tyr Gly Glu Asn Ile Gly Tyr Ser Glu Lys
        275                 280                 285

Asp Arg Phe Gln Gly Arg Phe Asp Val Lys Ile Glu Val Lys Ser
    290                 295                 300

<210>    8
<211>    305
<212>    PRT
<213>    Artificial Sequence

<220>
<223>    Chicken ADP-ribosyl Cyclase


<400>    8
Met Ala Arg Gly Lys Ala Asn Asp Gly Asp Gly Asn Trp Lys Lys Phe
1               5                   10                  15

Ile Trp Asn Ser Glu Lys Lys Glu Leu Leu Gly Arg Thr Gly Gly Ser
            20                  25                  30

Trp Phe Lys Ile Leu Leu Phe Tyr Val Ile Phe Tyr Gly Cys Leu Ala
        35                  40                  45

Gly Ile Phe Ile Gly Thr Ile Gln Val Met Leu Leu Thr Val Ser Glu
    50                  55                  60

Phe Glu Pro Lys Tyr Gln Asp Arg Val Ala Pro Pro Gly Leu Thr Gln
65                  70                  75                  80

Val Pro Gln Val Gln Lys Thr Glu Ile Ser Phe Thr Val Asn Asp Pro
                85                  90                  95

Lys Ser Tyr Asp Pro Tyr Val Lys Asn Leu Glu Gly Phe Leu Asn Lys
            100                 105                 110

Tyr Ser Ala Gly Glu Gln Thr Asp Asn Ile Val Phe Gln Asp Cys Gly
        115                 120                 125

Asp Ile Pro Thr Asp Tyr Lys Glu Arg Gly Pro Tyr Asn Asp Ala Gln
    130                 135                 140

Gly Gln Lys Lys Val Cys Lys Phe Lys Arg Glu Trp Leu Glu Asn Cys
145                 150                 155                 160

Ser Gly Leu Gln Asp Asn Thr Phe Gly Tyr Lys Asp Gly Lys Pro Cys
                165                 170                 175

Ile Leu Val Lys Leu Asn Arg Ile Ile Gly Phe Lys Pro Lys Ala Pro
            180                 185                 190

Glu Asn Glu Ser Leu Pro Ser Asp Leu Ala Gly Lys Tyr Asn Pro Tyr
        195                 200                 205

Leu Ile Pro Val His Cys Val Ala Lys Arg Asp Glu Asp Ala Asp Lys
    210                 215                 220

Ile Gly Met Val Glu Tyr Tyr Gly Met Gly Gly Tyr Pro Gly Phe Ala
225                 230                 235                 240

Leu Gln Tyr Tyr Pro Tyr Tyr Gly Arg Leu Leu Gln Pro Gln Tyr Leu
                245                 250                 255

Gln Pro Leu Val Ala Val Gln Phe Thr Asn Leu Thr Tyr Asp Val Glu
            260                 265                 270

Val Arg Val Glu Cys Lys Glu Tyr Gly Gln Asn Ile Gln Tyr Ser Asp
        275                 280                 285

Lys Asp Arg Phe Gln Gly Arg Phe Asp Ile Lys Phe Asp Ile Lys Ser
    290                 295                 300

Ser
305


<210>    9
<211>    133
<212>    PRT
<213>    Artificial Sequence

<220>
<223>    Cattle ADP-ribosyl Cyclase


<400>    9
Met Ala Arg Gly Lys Ala Lys Glu Glu Gly Ser Trp Lys Lys Phe Ile
  1               5                  10                  15

Trp Asn Ser Glu Lys Lys Glu Phe Leu Gly Arg Thr Gly Gly Ser Trp
             20                  25                  30

Phe Lys Ile Leu Leu Phe Tyr Val Ile Phe Tyr Gly Cys Leu Ala Gly
         35                  40                  45

Ile Phe Ile Gly Thr Ile Gln Val Met Leu Leu Thr Ile Ser Glu Phe
     50                  55                  60

Lys Pro Thr Tyr Gln Asp Arg Val Ala Pro Pro Gly Leu Thr Gln Ile
 65                  70                  75                  80

Pro Gln Ile Gln Lys Thr Glu Ile Ala Phe Arg Pro Asn Asp Pro Lys
                 85                  90                  95

Ser Tyr Met Thr Tyr Val Asp Asn Ile Asp Asn Phe Leu Lys Lys Tyr
            100                 105                 110

Ser Ala Leu Val Asp Leu Val Phe Ser Gln Val Lys Thr Met Asp Asn
        115                 120                 125

Lys Arg Met Asn Thr
    130


<210>    10
<211>    303
<212>    PRT
<213>    Artificial Sequence

<220>
<223>    Chimpanzee ADP-ribosyl Cyclase


<400>    10
Met Ala Arg Gly Lys Ala Lys Glu Glu Gly Ser Trp Lys Lys Phe Ile
  1               5                  10                  15

Trp Asn Ser Glu Lys Lys Glu Phe Leu Gly Arg Thr Gly Gly Ser Trp
             20                  25                  30

Phe Lys Ile Leu Leu Phe Tyr Val Ile Phe Tyr Gly Cys Leu Ala Gly
        35                  40                  45

Ile Phe Ile Gly Thr Ile Gln Val Met Leu Leu Thr Ile Ser Glu Phe
    50                  55                  60

Lys Pro Thr Tyr Gln Asp Arg Val Ala Pro Pro Gly Leu Thr Gln Ile
65                  70                  75                  80

Pro Gln Ile Gln Lys Thr Glu Ile Ser Phe Arg Pro Asn Asp Pro Lys
                85                  90                  95

Ser Tyr Glu Ala Tyr Val Leu Asn Ile Val Arg Phe Leu Glu Lys Tyr
            100                 105                 110

Lys Asp Ser Ala Gln Arg Asp Asp Met Ile Phe Glu Asp Cys Gly Asp
        115                 120                 125

Val Pro Ser Glu Pro Lys Glu Arg Gly Asp Phe Asn His Glu Arg Gly
    130                 135                 140

Glu Arg Lys Val Cys Arg Phe Lys Leu Glu Trp Leu Gly Asn Cys Ser
145                 150                 155                 160

Gly Leu Asn Asp Glu Thr Tyr Gly Tyr Lys Glu Gly Lys Pro Cys Ile
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Val Met Lys Tyr Asn Pro Asn Val
        195                 200                 205

Leu Pro Val Gln Cys Thr Gly Lys Arg Asp Glu Asp Lys Asp Lys Ile
    210                 215                 220

Gly Asn Val Glu Tyr Phe Gly Leu Gly Asn Ser Pro Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Leu Ala Val Gln Phe Thr Asn Leu Thr Met Asp Thr Glu Ile
            260                 265                 270

Arg Ile Glu Cys Lys Ala Tyr Gly Glu Asn Ile Gly Tyr Ser Glu Lys
        275                 280                 285

Asp Arg Phe Gln Gly Arg Phe Asp Val Lys Ile Glu Val Lys Ser
    290                 295                 300

<210>   11
<211>   303
<212>   PRT
<213>   Artificial Sequence

<220>
<223>   Horse ADP-ribosyl Cyclase

<400>    11
Met Ala Arg Gly Lys Ala Lys Glu Glu Gly Ser Trp Lys Lys Phe Ile
1               5                   10                  15

Trp Asn Ser Glu Lys Lys Glu Phe Leu Gly Arg Thr Gly Gly Ser Trp
            20                  25                  30

Phe Lys Ile Leu Leu Phe Tyr Val Ile Phe Tyr Gly Cys Leu Ala Gly
        35                  40                  45

Ile Phe Ile Gly Thr Ile Gln Val Met Leu Leu Thr Ile Ser Glu Phe
    50                  55                  60

Lys Pro Thr Tyr Gln Asp Arg Val Ala Pro Pro Gly Leu Thr Gln Ile
65                  70                  75                  80

Pro Gln Ile Gln Lys Thr Glu Ile Ser Phe Arg Pro Asn Asp Pro Lys
                85                  90                  95

Ser Tyr Glu Ala Tyr Val Leu Asn Ile Val Arg Phe Leu Glu Lys Tyr
            100                 105                 110

Lys Asp Ser Ala Gln Lys Asp Asp Met Ile Phe Glu Glu Cys Gly Ser
        115                 120                 125

Val Pro Ser Glu Leu Lys Glu Arg Gly Glu Phe Asn Asn Glu Arg Gly
    130                 135                 140

Glu Arg Lys Val Cys Arg Phe Lys Leu Glu Trp Leu Gly Asn Cys Ser
145                 150                 155                 160

Gly Ile Asn Asp Glu Thr Tyr Gly Tyr Lys Glu Gly Lys Pro Cys Val
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Val Met Lys Tyr Ser Pro Tyr Val
        195                 200                 205

Leu Pro Val Gln Cys Thr Gly Lys Arg Asp Glu Asp Lys Glu Lys Ile
    210                 215                 220

Gly Asn Val Glu Tyr Phe Gly Leu Gly Gly Tyr Pro Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Leu Ala Val Gln Phe Thr Asn Leu Thr Met Asp Thr Glu Ile
            260                 265                 270

Arg Ile Glu Cys Lys Ala Tyr Gly Glu Asn Ile Gly Tyr Ser Asp Lys
        275                 280                 285

Asp Arg Phe Gln Gly Arg Phe Asp Val Lys Ile Glu Val Lys Ser
    290                 295                 300

<210>    12

<211>    304
<212>    PRT
<213>    Artificial Sequence

<220>
<223>    Frog ADP-ribosyl Cyclase


<400>    12
Met Ala Arg Asp Lys Ala Lys Glu Thr Asp Gly Gly Trp Arg Lys Phe
1               5                   10                  15

Ile Trp Asn Pro Asp Lys Lys Glu Phe Leu Gly Arg Thr Gly Gly Ser
            20                  25                  30

Trp Phe Lys Ile Leu Leu Phe Tyr Leu Ile Phe Tyr Gly Cys Leu Ala
        35                  40                  45

Gly Ile Phe Ile Gly Thr Ile Gln Val Leu Leu Leu Thr Ile Ser Glu
    50                  55                  60

Tyr Glu Pro Lys Tyr Gln Asp Arg Val Ala Pro Pro Gly Leu Thr Gln
65                  70                  75                  80

Val Pro Lys Ala Val Lys Thr Glu Ile Asn Phe Ser Pro Asn Asp Pro
                85                  90                  95

Asp Ser Tyr Asn Asp Tyr Val Gln Ser Met Glu Lys Phe Ile Ser Lys
            100                 105                 110

Tyr Ser Asn Glu Asn Gln Val Ser Asp Lys Phe Glu Asp Cys Gly Thr
        115                 120                 125

Met Pro Gly Gln Tyr Arg Glu Arg Gly Gly Leu Asn Lys Asp Gly Gly
    130                 135                 140

Gln Lys Lys Ser Cys Val Phe Arg Arg Gln Trp Leu Gln Asn Cys Ser
145                 150                 155                 160

Gly Ile Asp Asp Gln Thr Phe Gly Phe Ala Glu Gly Lys Pro Cys Val
                165                 170                 175

Ile Val Lys Leu Asn Arg Ile Val Ala Phe Lys Pro Val Pro Pro Gln
            180                 185                 190

Asn Asn Ser Leu Pro Pro Glu Met Thr Ala Asn Tyr Asn Pro Tyr Val
        195                 200                 205

Ile Pro Ile His Cys Gln Gly Lys Arg Asp Glu Asp Ile Pro Asn Ile
    210                 215                 220

Arg Glu Val Lys Tyr Tyr Gly Met Gly Gly Phe Ala Gly Phe Pro Leu
225                 230                 235                 240

Asn Tyr Tyr Pro Tyr Tyr Gly Lys Leu Leu Gln Pro Glu Tyr Leu Gln
                245                 250                 255

Pro Leu Ile Ala Val Gln Phe Thr Asn Leu Thr Phe Asn Thr Glu Ile
            260                 265                 270

Arg Ile Glu Cys Lys Ala Tyr Gly Glu Asn Ile Asp Tyr His Asp Lys
        275                 280                 285

Asp Arg Phe Gln Gly Arg Phe Asp Ile Lys Phe Asp Ile Lys Ser Ser
    290                 295                 300

<210>    13
<211>    303
<212>    PRT
<213>    Artificial Sequence

<220>
<223>    Goat ADP-ribosyl Cyclase

<400>    13
Met Ala Arg Gly Lys Ala Lys Glu Glu Gly Ser Trp Lys Lys Phe Ile
  1               5                  10                  15

Trp Asn Ser Glu Lys Lys Glu Phe Leu Gly Arg Thr Gly Gly Ser Trp
             20                  25                  30

Phe Lys Ile Leu Leu Phe Tyr Val Ile Phe Tyr Gly Cys Leu Ala Gly
         35                  40                  45

Ile Phe Ile Gly Thr Ile Gln Val Met Leu Leu Thr Ile Ser Glu Phe
     50                  55                  60

Lys Pro Thr Tyr Gln Asp Arg Val Ala Pro Pro Gly Leu Thr Gln Ile
 65                  70                  75                  80

Pro Gln Ile Gln Lys Thr Glu Ile Ala Phe Arg Pro Asn Asp Pro Lys
                 85                  90                  95

Ser Tyr Met Thr Tyr Val Asp Asn Ile Asp Asn Phe Leu Lys Lys Tyr
            100                 105                 110

Arg Asp Ser Ala Gln Lys Asp Asp Met Ile Phe Glu Asp Cys Gly Asn
        115                 120                 125

Val Pro Ser Glu Leu Lys Asp Arg Gly Glu Phe Asn Asn Glu Gln Gly
    130                 135                 140

Glu Arg Lys Val Cys Arg Phe Lys Leu Glu Trp Leu Gly Asn Cys Ser
145                 150                 155                 160

Gly Ile Asn Asp Glu Thr Tyr Gly Tyr Arg Glu Gly Lys Pro Cys Val
                165                 170                 175

Ile Ile Lys Leu Asn Arg Val Leu Gly Phe Lys Pro Lys Pro Pro Lys
            180                 185                 190

Asn Glu Ser Leu Glu Thr Tyr Pro Val Met Lys Tyr Asn Pro Tyr Val
        195                 200                 205

Leu Pro Val Gln Cys Thr Gly Lys Arg Asp Glu Asp Lys Glu Lys Val
    210                 215                 220

Gly Ser Ile Glu Tyr Phe Gly Leu Gly Gly Tyr Pro Gly Phe Pro Leu
225                 230                 235                 240

Gln Tyr Tyr Pro Tyr Tyr Gly Lys Leu Leu Gln Pro Lys Tyr Leu Gln
                245                 250                 255

Pro Leu Leu Ala Val Gln Phe Thr Asn Leu Thr Met Asp Thr Glu Ile
                260                 265                 270

Arg Ile Glu Cys Lys Ala Tyr Gly Glu Asn Ile Gly Tyr Ser Glu Lys
        275                 280                 285

Asp Arg Phe Gln Gly Arg Phe Asp Val Lys Ile Glu Val Lys Ser
    290                 295                 300

<210>    14
<211>    319
<212>    PRT
<213>    Artificial Sequence

<220>
<223>    Turkey ADP-ribosyl Cyclase

<400>    14
Met Thr Val Leu Trp Lys Lys Phe Pro Asn Ala Ser Asp Val Thr Ala
  1               5                  10                  15

Ile Gly Phe Thr Leu Pro Gly Tyr Gln Arg Val Phe Phe Asn Ser Trp
             20                  25                  30

Arg Thr Ala Leu Leu His Gln Ala Leu Pro Ala Ser Pro Gly Cys Val
         35                  40                  45

Lys Ile Leu Leu Phe Tyr Val Ile Phe Tyr Gly Cys Leu Ala Gly Ile
     50                  55                  60

Phe Ile Gly Thr Ile Gln Val Met Leu Leu Thr Val Ser Glu Phe Glu
 65                  70                  75                  80

Pro Lys Tyr Gln Asp Arg Val Ala Pro Pro Gly Leu Thr Gln Val Pro
                 85                  90                  95

Gln Val Gln Lys Thr Glu Ile Ser Phe Thr Val Ser Asp Pro Lys Ser
            100                 105                 110

Tyr Asp Pro Tyr Val Lys Asn Leu Glu Gly Phe Leu Ser Lys Tyr Ser
        115                 120                 125

Ala Gly Glu Gln Thr Asp Asn Ile Val Phe Gln Asp Cys Gly Asp Val
    130                 135                 140

Pro Met Asp Tyr Lys Glu Arg Gly Pro Tyr Asn Asp Asp Gln Gly Gln
145                 150                 155                 160

Lys Lys Val Cys Lys Phe Lys Arg Glu Trp Leu Glu Asn Cys Ser Gly
                165                 170                 175

Leu Gln Asp Asn Thr Phe Gly Tyr Lys Glu Gly Lys Pro Cys Ile Leu
            180                 185                 190

Val Lys Leu Asn Arg Ile Ile Gly Phe Lys Pro Lys Ala Pro Glu Asn
        195                 200                 205

Glu Ser Leu Pro Leu Gly Leu Ala Gly Lys Tyr Asn Pro Phe Leu Ile

210                 215                 220

Pro Val His Cys Val Ala Lys Arg Asp Glu Asp Ser Asp Lys Ile Gly
225                 230                 235                 240

Thr Val Glu Tyr Tyr Gly Met Gly Gly Tyr Pro Gly Phe Ala Leu Gln
                245                 250                 255

Tyr Tyr Pro Tyr Tyr Gly Lys Leu Leu Gln Pro His Tyr Leu Gln Pro
            260                 265                 270

Leu Val Ala Val Gln Phe Thr Asn Leu Thr Tyr Asp Val Glu Val Arg
        275                 280                 285

Val Glu Cys Arg Ala Tyr Gly Gln Asn Ile Gln Tyr Ser Asp Lys Asp
    290                 295                 300

Arg Phe Gln Gly Arg Phe Asp Ile Lys Phe Asp Ile Arg Ser Ser
305                 310                 315


<210>    15
<211>    306
<212>    PRT
<213>    Artificial Sequence

<220>
<223>    Guinea Pig ADP-ribosyl Cyclase


<400>    15
Met Ala Arg Gly Lys Ala Lys Glu Glu Gly Ser Trp Lys Lys Phe Ile
1               5                   10                  15

Trp Asn Ser Glu Lys Lys Glu Phe Leu Gly Arg Thr Gly Gly Ser Trp
            20                  25                  30

Phe Lys Ile Leu Leu Phe Tyr Val Ile Phe Tyr Gly Cys Leu Ala Gly
        35                  40                  45

Ile Phe Ile Gly Thr Ile Gln Val Met Leu Leu Thr Ile Ser Glu Leu
    50                  55                  60

Lys Pro Thr Tyr Gln Asp Arg Val Ala Pro Pro Gly Leu Thr Gln Ile
65                  70                  75                  80

Pro Gln Ile Gln Lys Thr Glu Ile Ser Phe Arg Pro Ala Asp Pro Lys
                85                  90                  95

Ser Tyr Glu Ala Tyr Val Leu Asn Ile Tyr Arg Phe Leu Glu Lys Tyr
            100                 105                 110

Lys Asp Ala Ala Gln Lys Asp Asp Met Ile Phe Glu Asp Cys Ser Thr
        115                 120                 125

Val Pro Ser Glu Pro Lys Glu Arg Gly Asp Phe Asn His Glu Arg Gly
    130                 135                 140

Glu Arg Lys Val Cys Arg Phe Lys Leu Glu Trp Leu Gly Asn Cys Ser
145                 150                 155                 160

Gly Gln Asn Asp Asp Ser Tyr Gly Tyr Arg Asp Gly Lys Pro Cys Ile
                165                 170                 175

Ile Ile Lys Leu Asn Arg Val Leu Gly Phe Lys Pro Lys Pro Pro Lys
            180                 185                 190

Asn Asp Ser Ser Glu Thr Val Glu Ile Tyr Ser Thr Met Lys Tyr Asn
        195                 200                 205

Pro Tyr Val Leu Pro Val Gln Cys Thr Gly Lys Arg Glu Glu Asp Lys
    210                 215                 220

Asp Lys Ile Gly Ser Val Glu Tyr Phe Gly Leu Gly Gly Tyr Ala Gly
225                 230                 235                 240

Phe Pro Leu Gln Tyr Tyr Pro Tyr Tyr Gly Lys Leu Leu Gln Pro Lys
                245                 250                 255

Tyr Leu Gln Pro Leu Leu Ala Val Gln Phe Thr Asn Leu Thr Thr Asp
            260                 265                 270

Thr Glu Val Arg Ile Glu Cys Lys Ala Tyr Gly Glu Asn Ile Gly Tyr
        275                 280                 285

Ser Glu Lys Asp Arg Phe Gln Gly Arg Phe Asp Val Lys Ile Glu Val
    290                 295                 300

Lys Ser
305

<210> 16
<211> 280
<212> PRT
<213> Artificial Sequence

<220>
<223> E.granulos ADP-ribosyl Cyclase

<400> 16
Met Val Arg Cys Ser Glu Arg Tyr Arg Asn Leu Gly Leu Ala Ile Phe
1               5                   10                  15

Asn Pro Lys Glu Lys Lys Phe Cys Gly Arg Thr Cys Arg Ser Trp Ala
            20                  25                  30

Leu Ile Phe Val Tyr Tyr Leu Ile Phe Tyr Ser Cys Leu Ala Gly Phe
        35                  40                  45

Trp Ile Gly Met Leu Ser Val Leu Ile Phe Ala Met Ile Asp Thr Thr
    50                  55                  60

Val Pro Ser Leu Thr Gly Met Gln Ser Leu Leu Lys Leu Asn Pro Gly
65                  70                  75                  80

Leu Gly Ile Leu Pro Pro Val Asp Ser Glu Gly Thr Leu Ile Gln Leu
                85                  90                  95

Thr Leu Phe Asp Ser Lys Gln Lys Gln Asp Tyr Leu Asn Phe Met Gln
            100                 105                 110

Ser Tyr Leu Met Gly Tyr Ser Asn Ile Ser Thr Asn Cys Asp Phe Glu
        115                 120                 125

Asn Gly Thr Arg Ile Asn Ser Ser Ile Leu Glu Pro Cys Glu Phe Pro
    130                 135                 140

Leu Ser Leu Leu Gly Pro Cys Ala Asp Pro Ala Asp Tyr Ile Asn Ser
145                 150                 155                 160

His Asn Asn Phe Cys Phe Tyr Leu Lys Leu Asn Lys Ile Tyr Gly Tyr
                165                 170                 175

Leu Pro Asp Ile Glu Gly Asn Lys Ile Pro Ile Gln Cys Gly Pro Ala
            180                 185                 190

Asn Ser Phe Asp Gly Ala Asn Leu Gly Gln Pro Val Tyr Tyr Pro Ser
        195                 200                 205

Val Arg Thr Ala Asn Gly Thr Phe Gly Tyr Phe Ser Ser Val Ala Phe
    210                 215                 220

Pro Tyr Leu Asn Gln Pro His Tyr Gln Val Pro Leu Leu Ala Val Thr
225                 230                 235                 240

Phe Pro Asp Ile Lys Pro Asn Thr Val Val Met Val Ser Cys Ala Val
                245                 250                 255

Leu Asn Val Lys Asp Gln Glu Pro Phe Arg Phe Asp Leu Ala Ile Asp
            260                 265                 270

Met Asp Arg Pro Ile Ala Leu Thr
        275                 280


<210>    17
<211>    232
<212>    PRT
<213>    Artificial Sequence

<220>
<223>    S.haematob ADP-ribosyl Cyclase


<400>    17
Met Gly Met Leu Ile Ile Ile Thr Gln Leu Ile Ile Ser Asn Asp Gln
  1               5                  10                  15

Pro Tyr Ile Thr Gly Met Asp Ser Pro Leu Ala Leu Ser Pro Gly Leu
            20                  25                  30

Gly Met Arg Pro Arg Asn Asp Phe Lys Thr Thr Leu Ile Ala Tyr Ala
        35                  40                  45

Ser Ser Asp Pro Gln Thr Tyr Met Pro Phe Val Gln Asp Ile Arg Thr
    50                  55                  60

Phe Leu Tyr Phe Tyr Glu Glu Val Asn Ile Gln Pro Gln Asp Gly Phe
 65                  70                  75                  80

Ala Thr Cys Asp Lys Ile Lys Ser Pro Asp Asp Val Asp Leu Val Cys
                85                  90                  95

Lys Phe Tyr Pro His Asp Met Gly Val Cys Val Lys Glu Asn Asn Phe
            100                 105                 110

Gly Tyr Asp Arg Ser Gln Pro Cys Val Ile Met Lys Ile Asn Lys Val

115                 120                 125

Tyr Gly Trp Leu Pro Asp Ile Val Asn Lys Thr Leu Ser Asn Asn Pro
    130                 135                 140

Leu Val Arg Cys His Gly Gln Asn Pro Gln Asp Leu Glu Asn Phe Gly
145                 150                 155                 160

Gln Val Leu Tyr Phe Pro Asn Ile Thr Val Asp Gly Lys Thr Tyr Gly
                165                 170                 175

Tyr Phe Ser His Leu Tyr Phe Pro Tyr Leu Met Gln Val Ala Tyr Arg
            180                 185                 190

Ser Pro Leu Val Ala Val Gln Phe Ala Ser Pro Lys Arg His Val Leu
        195                 200                 205

Leu Met Val Arg Cys Glu Leu Phe Asn Val Arg Asn Pro Gly Asp Pro
    210                 215                 220

Met Asp Phe Glu Ile Leu Val Asp
225                 230


<210>    18
<211>    190
<212>    PRT
<213>    Artificial Sequence

<220>
<223>    T.spiralis ADP-ribosyl Cyclase


<400>    18
Met Gly Tyr Gln Pro Trp Leu Arg Asp Asp Pro Glu Ser Thr Leu Ile
  1               5                  10                  15

Tyr Phe Asn Arg Ser Glu Pro Ser Thr Tyr Lys Asn Asn Ser Ser Asn
             20                  25                  30

Arg Glu Asn Thr His Ile Ala Cys Ala Phe Asp Leu Ile Glu His Phe
         35                  40                  45

Glu Ser Gln Gly Cys Gly Glu Lys Asp Asp Phe Gly Phe Lys Asn Ala
     50                  55                  60

Thr Pro Cys Ile Val Leu Thr Leu Asn Lys Leu Ile Gly Trp Glu Pro
 65                  70                  75                  80

Val Ala Tyr Pro Lys Asp Ser Ala Pro Asp Ala Ile Lys Asp His Tyr
                 85                  90                  95

Asn Tyr Ser Thr Pro Asp Val Ala Ile Ala Cys Glu Gly Glu Phe Pro
            100                 105                 110

Val Asp Gln Glu His Ile Gly Pro Leu Gln Tyr Ile Pro Pro Thr Gly
        115                 120                 125

Ile Pro His Lys Phe Phe Pro Tyr Arg Val Met Pro Asn Tyr His Gln
    130                 135                 140

Pro Phe Ala Leu Val Lys Phe Val Gly Pro Pro Lys Gly Ile Leu Ile
145                 150                 155                 160

Glu Val Glu Cys Lys Ala Tyr Ala Tyr Asn Ile Met His Asp Arg Ser
                165                 170                 175

Tyr Arg Leu Gly Met Val His Phe Glu Leu Leu Ile Asp Ser
            180                 185                 190

<210>    19
<211>    345
<212>    PRT
<213>    Artificial Sequence

<220>
<223>    Drosophila ADP-ribosyl Cyclase

<400>    19
Met Pro Glu Asp Val Met Val Pro Ser Gly Asn Tyr Lys Val Arg Lys
  1               5                  10                  15

Arg Phe Arg Asn Glu Glu His Arg Arg Ser Lys Lys Asp Met Pro Trp
            20                  25                  30

Thr Lys Gln Ile Leu Asp Leu Asn Glu Asn Arg Phe Phe Gly Arg Thr
        35                  40                  45

Ala Trp Ala Trp Cys Arg Ile Val Ser Phe Tyr Leu Leu Leu Tyr Leu
    50                  55                  60

Leu Ile Phe Leu Ile Leu Leu Cys Leu Tyr Leu Ile Phe Arg Phe Tyr
 65                  70                  75                  80

Phe Ile Gln Lys Asp Arg Pro Ser Ile Leu Lys Glu Ala Pro Gly Leu
                85                  90                  95

Ser Ile Val Pro Arg Asn Glu Ser Thr Ile Thr Phe Tyr Tyr Asn Gln
            100                 105                 110

Met Pro Asp Ile Tyr Pro Leu Cys Asp Arg Ile Asp Glu Phe Leu Glu
        115                 120                 125

Lys Leu Asp Asp Glu Ala Phe Glu Tyr Phe His Glu Cys Asn Gly Asp
    130                 135                 140

Lys Leu Trp Gly Tyr Asn Glu Lys Lys Pro Cys Val Phe Val Lys Leu
145                 150                 155                 160

Asn Lys Ile Phe Gly Phe Lys Pro Glu Val Tyr Thr Ser Pro Thr Glu
                165                 170                 175

Leu Pro Ser Glu Ala Pro Pro Glu Leu Thr Thr Ile Met Gly Lys Phe
            180                 185                 190

Lys Gly His Asp Arg Ile Trp Leu Thr Cys Glu Leu Ser Gln Gly Lys
        195                 200                 205

Leu Pro Lys Ile Val Tyr Ile Pro Gly Pro Tyr Phe Asp Thr Glu Glu
    210                 215                 220

Leu Ala Gly Val Ser Arg Val Val Ala Leu Gln Leu Thr Glu Met Pro
225                 230                 235                 240

Glu Asn Lys Glu Ile Phe Val Ser Cys Arg Val Trp Ala Lys Asn Ile
                245                 250                 255

Lys Ile Asp Leu Lys Gln Thr Gly Arg Gly Asn Ala Lys Phe Ser Met
            260                 265                 270

Lys Met Lys Val Lys Lys Ser Asn Asn Asn Pro Pro Asp Asn Leu Glu
        275                 280                 285

Thr Arg Pro Thr Lys Phe Lys Val Trp Lys Pro Asn Gln Asp Gln Gln
    290                 295                 300

Arg Ile Phe Tyr Asp Glu Asp Leu Gln Leu Pro Asp Val Pro Asp Leu
305                 310                 315                 320

Glu Ala Glu Asp Asp Gly Val Lys Thr Asp Thr Arg Ser Ala Ile Pro
                325                 330                 335

Thr Glu Pro Pro Asp Lys Leu Lys Arg
            340                 345

<210>    20
<211>    306
<212>    PRT
<213>    Artificial Sequence

<220>
<223>    Zebrafish Type A ADP-ribosyl Cyclase

<400>    20
Met Pro Ala Asn Lys Asp Gly Asp Gly Gly Trp Lys Ser Phe Ile Trp
  1               5                  10                  15

Asn Ser Asp Lys Lys Glu Phe Leu Gly Arg Thr Gly Cys Ser Trp Leu
            20                  25                  30

Lys Ile Phe Ile Phe Tyr Val Ile Phe Tyr Gly Cys Leu Ala Gly Ile
        35                  40                  45

Phe Ile Gly Thr Ile Gln Ala Met Leu Leu Thr Leu Ser Asn Tyr Lys
    50                  55                  60

Pro Thr Tyr Gln Asp Arg Val Ala Pro Pro Gly Leu Ser His Ser Pro
 65                  70                  75                  80

Arg Pro Asp Lys Ala Glu Ile Asn Tyr Asn Ile Asn Asp Glu Ser Thr
                85                  90                  95

Tyr Leu Pro Tyr Val Asn His Ile Asp Ala Phe Leu Lys Ala Tyr Asn
            100                 105                 110

Glu Asp Val Gln Lys Asp Asp Thr Lys Phe Glu Glu Cys Gly Asp Lys
        115                 120                 125

Pro Gln Phe Tyr Thr Asp Arg Gly Glu Leu Glu Ser Asp Asn Gly Val
    130                 135                 140

Arg Lys Ala Cys Arg Phe Arg Arg Glu Trp Leu Gly Glu Cys Ser Gly
145                 150                 155                 160

Gln Lys Asp Glu Lys Leu Lys Asn Tyr Gly Phe Asp Asp Gly Gln Pro

165                 170                 175

Cys Leu Ile Val Lys Leu Asn Arg Ile Val Asn Phe Met Pro Arg Pro
            180                 185                 190

Pro Ala Ser Asn Asp Ser Ile Pro Glu Ala Val Arg Pro Lys Leu Gln
        195                 200                 205

Gly Asn Val Ile Pro Ile His Cys Ser Ser Lys Arg Glu Glu Glu Ala
    210                 215                 220

Asn Leu Leu Gly Gln Ile Lys Tyr Phe Gly Leu Gly Thr Gly Phe Pro
225                 230                 235                 240

Leu Gln Tyr Tyr Pro Tyr Tyr Gly Lys Leu Leu Gln Pro Gln Tyr Leu
                245                 250                 255

Gln Pro Leu Val Ala Ile Lys Phe Tyr Asn Ile Thr Thr Asp Val Asp
            260                 265                 270

Val Arg Val Glu Cys Lys Val Tyr Gly Glu Asn Ile Asp Tyr Ser Glu
        275                 280                 285

Lys Asp Arg Ser Gln Gly Arg Phe Asp Ile Lys Phe Thr Ile Lys Thr
    290                 295                 300

Lys Ser
305


<210>    21
<211>    302
<212>    PRT
<213>    Artificial Sequence

<220>
<223>    Zebrafish Type B ADP-ribosyl Cyclase


<400>    21
Met Pro Ala Gln Asn Lys Asp Asp Gly Gly Trp Lys Lys Phe Val Trp
1               5                   10                  15

Asn Ser Glu Lys Lys Glu Phe Leu Gly Arg Thr Gly Gly Ser Trp Ala
            20                  25                  30

Lys Ile Phe Leu Phe Tyr Leu Ile Phe Tyr Gly Cys Leu Ala Gly Ile
        35                  40                  45

Phe Ile Gly Thr Ile Gln Ile Leu Leu Leu Thr Leu Ser Asp Tyr Lys
    50                  55                  60

Pro Thr Trp Gln Asp Arg Val Ala Pro Pro Gly Leu Thr His Phe Pro
65                  70                  75                  80

Arg Ser Asp Lys Ser Glu Ile Ala Ile Asn Leu Asp Asp Glu Val Ser
                85                  90                  95

Phe Leu Asn Tyr Val Lys Val Met Arg Glu Phe Leu Thr Ser Tyr Asp
            100                 105                 110

Gln Glu Lys Gln Leu Asp Asn Met Gln Phe Glu Asn Cys Gly Glu Ser
        115                 120                 125

Pro Leu Asp Tyr Lys Asn Arg Gly Asp Leu Glu Ser Asp Val Gly Val
    130                 135                 140

Arg Arg Ala Cys Gln Phe Ser Arg Glu Trp Leu Gly Pro Cys Ser Gly
145                 150                 155                 160

Leu Asp Asp Pro Tyr Phe Gly Phe Lys Glu Gly Lys Pro Cys Leu Ile
                165                 170                 175

Ala Lys Leu Asn Arg Ile Val Asn Phe Arg Pro Lys Pro Pro Val Ser
            180                 185                 190

Asn Glu Ser Ile Pro Glu Glu Val Gln His Lys Val Gln Pro Tyr Leu
        195                 200                 205

Ile Pro Ile His Cys Thr Asn Lys Lys Glu Glu Asp Ala Gly Lys Leu
    210                 215                 220

Gly Glu Val Arg Tyr Tyr Gly Phe Gly Gly Gly Phe Pro Leu Gln Tyr
225                 230                 235                 240

Tyr Pro Tyr Tyr Gly Lys Leu Leu His Pro Gln Tyr Leu Gln Pro Leu
                245                 250                 255

Val Ala Ile Gln Phe Leu Asn Ile Thr Pro Asn Thr Asp Met Arg Ile
            260                 265                 270

Glu Cys Lys Val Tyr Gly Glu Asn Ile Tyr Tyr His Asp Lys Asp Arg
        275                 280                 285

Tyr Gln Gly Arg Phe Asp Val Lys Phe Asn Ile Lys Lys Ser
    290                 295                 300

<210>    22
<211>    25
<212>    RNA
<213>    Artificial Sequence

<220>
<223>    ADPRC siRNA

<400>    22
cccaagaaug aauccuugga gacuu                                          25